# EUROPEAN PATENT APPLICATION

(11) **EP 2 175 017 A1**
(43) Date of publication of application: **14.04.2010**
(21) Application number: 08777718.1
(22) Date of filing: 30.06.2008
(51) Int. Cl.: C12N 15/09, A61K 39/104, A61K 39/395, A61P 31/04, C07K 14/21, C07K 16/12, C12N 5/10, G01N 33/569, C12P 21/08

(54) **PSEUDOMONAS AERUGINOSA OUTER MEMBRANE PROTEIN PA4710**

(30) Priority: 29.06.2007 JP 2007171680
(71) Applicant: Meiji Seika Kaisha Ltd., Tokyo 104-8002 (JP)
(72) Inventor: TANAKA, Jiro, Yokohama-shi Kanagawa 222-8567 (JP); NAGASO, Hiroshi, Yokohama-shi Kanagawa 222-8567 (JP); KUMAGAI, Masashi, Yokohama-shi Kanagawa 222-8567 (JP); OTSUKA, Keiko, Yokohama-shi Kanagawa 222-8567 (JP); AKABANE, Hirotomo, Yokohama-shi Kanagawa 222-8567 (JP); SUZUKI, Takahisa, Yokohama-shi Kanagawa 222-8567 (JP)
(74) Representative: Cohausz & Florack
(86) International application number: PCT/JP2008/061867
(87) International publication number: WO 2009/005040

(57) **Abstract**

An object is to provide: a protein antigen or a peptide antigen usable as a vaccine composition which has an ability to practically prevent or treat a Pseudomonas aeruginosa infection, and which can cope with the diversity of clinical isolates derived from patients with a Pseudomonas aeruginosa infection; and an antibody directed against the antigen. The present invention provides a protein antigen or a peptide antigen and an antibody directed against these, which are for use in diagnosis, prevention, or treatment of a disease associated with Pseudomonas aeruginosa. According to the present invention, a protein or a peptide derived from a Pseudomonas aeruginosa-outer membrane protein PA4710, and an antibody directed against these are provided, which are for use in diagnosis, prevention, or treatment of a disease associated with Pseudomonas aeruginosa.

SEQUENCE LISTING

<110>  Meiji Seika Kaisha Ltd.

<120>  Pseudomonas aeruginosa outer membrane protein PA4710

<130>  PM1904

<150>  JP 2007-171680
<151>  2007-07-29

<160>  39

<170>  PatentIn version 3.1

<210>  1
<211>  2295
<212>  DNA
<213>  Pseudomonas aeruginosa

<400>  1
atgccgctct ccccgccctt cgccctgcgc ccctgcctgg ccctgctgtt gctcagccct      60

tccctggccc tggcggggaa cgccgtcccg ctgaccccga ccaccatcac cgccacccgt     120

accgagcagg cagtggattc ggtgccaagc accgtcagcg tgcagacccg cgaacaactg     180

gaccggcaga acgtcaacaa catcaaggaa ctggtgcgct acgaaccggg agtctcggtc     240

ggcggcgccg gccagcgtgc cgggatcacc ggctacaaca tccgcggcat cgacgggaac     300

cgcatcctta cgcagatcga cggggtcgaa ctgcccaacg acttcttcag cggcccctac     360

gcgcagaccc accgcaacta cgtcgatccg gacatcgtaa agcgcgtgga gatccttcgc     420

ggcccggcct cggcgctgta cggcagcaac gccatcggcg gcgcggtgag ctacttcacc     480

ctcgacccgt cggacatcat caaggacggc aaggacgtcg gcgcccggct gaaggccggc     540

tacgagtcgg ccagccactc ctggttgacc tcggccaccg tcgccggccg cgccgacgac     600

ttcgacggcc tgctgcatta tggctaccgc cagggccacg agaccgaatc caacggcggc     660

cacggcggca ccgggctctc gcgcagcgaa gccaacccgg aagacgccga cagctacagc     720

ctgctcggca agctgggctg gaactacgcc gagggcagcc gcttcgggct ggtcttcgag     780

aagtacaaga gcgacgtcga taccgaccag aagagcgcct atggcggccc gtacgacaag     840

ggcaagccgg ccatcccgcc gagcatgctg ccgggcggca tgtaccagtg gcgcaagggc     900

aacgacaccc tgactcgcga gcgctacggc ctggagcacc atttcctgct cgacagccag     960

gtcgccgatc gcatccagtg gagcctgaac taccagttgg cgaagaccga ccaggcgacc    1020

cgcgagttct actacccgat cacccgcaag gtcctgcgca cccgcgacac tacctacaag    1080

gaacgcctgt gggtcttcga cagccagttg gacaagagct tcgccatcgg cgagaccgag    1140

cacctgctga gctacgggat caatctcaag caccagaagg tcaccggcat gcgcagcggc    1200

accggcacca acctggacac cggcgcggac agcccgcgcg atgccctgga acgcagcagc    1260

gactttcccg atccgacggt gaagacctac gccctgttcg cccaggacag catcagctgg    1320

aacgactgga ccttcactcc cggcctgcgt tacgactaca cgcgcatgga gccgcacatc    1380

accgacgagt tcctgcgcac catgaagcag agccagaaca ccgcggtcga cgagtcggac    1440

aagaaatggc accgggtttc gcccaagttc ggcgtgacct acgacttcgc ccagcactac 1500

acctggtacg gccaatacgc ccagggcttc cgcacgccca ccgccaaggc gctgtacggt 1560

cgattcgaga acctgcaggc gggctaccac atcgagccta accccaacct caagccggaa 1620

aagagccaga gcttcgagac cgggttgcgc ggcaagttcg acgaaggcag cttcggtgta 1680

gcggtgttct acaacaaata tcgcgacttc atcgacgaag acgccctgaa taccgatagc 1740

accggcggca acggccagac cttccagtcc aacaacatcg agcgggcggt gatcaagggc 1800

gtcgagctca agggccgcct ggagctgggc gccttcggcg cgccgcaggg gctctacacc 1860

cagggcagcg tggcctacgc ctacggtcgc aacaaggaca acggcgagcc gatcaacagc 1920

gtcaacccac tcaccggagt gttcggcctg ggctacgacg aagcagacgg caactacggc 1980

gggctgctca gctggaccct ggtcaaacgc aaggatcgcg tcgacgacag caccttccac 2040

accccggatg gcaccgccag ccagttcaag accccgggct tcggcgtcct cgacctcagc 2100

gcctactaca ggctgagcaa ggacctgacc ctcaacgccg gtctctacaa cctgaccgac 2160

aagaaatact ggctgtggga tgacgtgcgc ggctacgaca gcgtcggcga ggcttcggcg 2220

ctggccccgg ccaacatcga ccgactgtcc cagccaggcc gcaatttcgc ggtcaacctg 2280

gtctgggaca tctga 2295

<210> 2
<211> 1755
<212> DNA
<213> Pseudomonas aeruginosa

<400> 2
tacgagtcgg ccagccactc ctggttgacc tcggccaccg tcgccggccg cgccgacgac 60

ttcgacggcc tgctgcatta tggctaccgc cagggccacg agaccgaatc caacggcggc 120

cacggcggca ccgggctctc gcgcagcgaa gccaacccgg aagacgccga cagctacagc 180

ctgctcggca agctgggctg gaactacgcc gagggcagcc gcttcgggct ggtcttcgag 240

aagtacaaga gcgacgtcga taccgaccag aagagcgcct atggcggccc gtacgacaag 300

ggcaagccgg ccatcccgcc gagcatgctg ccgggcggca tgtaccagtg gcgcaagggc 360

aacgacaccc tgactcgcga gcgctacggc ctggagcacc atttcctgct cgacagccag 420

gtcgccgatc gcatccagtg gagcctgaac taccagttgg cgaagaccga ccaggcgacc 480

cgcgagttct actacccgat cacccgcaag gtcctgcgca cccgcgacac tacctacaag 540

gaacgcctgt gggtcttcga cagccagttg gacaagagct tcgccatcgg cgagaccgag 600

cacctgctga gctacgggat caatctcaag caccagaagg tcaccggcat gcgcagcggc 660

accggcacca acctggacac cggcgcggac agcccgcgcg atgccctgga acgcagcagc 720

gactttcccg atccgacggt gaagacctac gccctgttcg cccaggacag catcagctgg 780

aacgactgga ccttcactcc cggcctgcgt tacgactaca cgcgcatgga gccgcacatc 840

accgacgagt tcctgcgcac catgaagcag agccagaaca ccgcggtcga cgagtcggac 900

aagaaatggc accgggtttc gcccaagttc ggcgtgacct acgacttcgc ccagcactac 960

acctggtacg gccaatacgc ccagggcttc cgcacgccca ccgccaaggc gctgtacggt 1020

cgattcgaga acctgcaggc gggctaccac atcgagccta accccaacct caagccggaa 1080

aagagccaga gcttcgagac cgggttgcgc ggcaagttcg acgaaggcag cttcggtgta 1140

gcggtgttct acaacaaata tcgcgacttc atcgacgaag acgccctgaa taccgatagc 1200

accggcggca acggccagac cttccagtcc aacaacatcg agcgggcggt gatcaagggc 1260

gtcgagctca agggccgcct ggagctgggc gccttcggcg cgccgcaggg gctctacacc 1320

cagggcagcg tggcctacgc ctacggtcgc aacaaggaca acggcgagcc gatcaacagc 1380

gtcaacccac tcaccggagt gttcggcctg ggctacgacg aagcagacgg caactacggc 1440

gggctgctca gctggaccct ggtcaaacgc aaggatcgcg tcgacgacag caccttccac 1500

accccggatg gcaccgccag ccagttcaag accccgggct tcggcgtcct cgacctcagc 1560

gcctactaca ggctgagcaa ggacctgacc ctcaacgccg gtctctacaa cctgaccgac 1620

aagaaatact ggctgtggga tgacgtgcgc ggctacgaca gcgtcggcga ggcttcggcg 1680

ctggccccgg ccaacatcga ccgactgtcc cagccaggcc gcaatttcgc ggtcaacctg 1740

gtctgggaca tctga 1755

<210> 3
<211> 764
<212> PRT
<213> Pseudomonas aeruginosa

<400> 3

Met Pro Leu Ser Pro Pro Phe Ala Leu Arg Pro Cys Leu Ala Leu Leu
1               5                   10                  15

Leu Leu Ser Pro Ser Leu Ala Leu Ala Gly Asn Ala Val Pro Leu Thr
            20                  25                  30

Pro Thr Thr Ile Thr Ala Thr Arg Thr Glu Gln Ala Val Asp Ser Val
        35                  40                  45

Pro Ser Thr Val Ser Val Gln Thr Arg Glu Gln Leu Asp Arg Gln Asn
    50                  55                  60

Val Asn Asn Ile Lys Glu Leu Val Arg Tyr Glu Pro Gly Val Ser Val
65                  70                  75                  80

Gly Gly Ala Gly Gln Arg Ala Gly Ile Thr Gly Tyr Asn Ile Arg Gly
                85                  90                  95

Ile Asp Gly Asn Arg Ile Leu Thr Gln Ile Asp Gly Val Glu Leu Pro
            100                 105                 110

Asn Asp Phe Phe Ser Gly Pro Tyr Ala Gln Thr His Arg Asn Tyr Val
        115                 120                 125

Asp Pro Asp Ile Val Lys Arg Val Glu Ile Leu Arg Gly Pro Ala Ser
    130                 135                 140

Ala Leu Tyr Gly Ser Asn Ala Ile Gly Gly Ala Val Ser Tyr Phe Thr
145                 150                 155                 160

Leu Asp Pro Ser Asp Ile Ile Lys Asp Gly Lys Asp Val Gly Ala Arg
                165                 170                 175

Leu Lys Ala Gly Tyr Glu Ser Ala Ser His Ser Trp Leu Thr Ser Ala
            180                 185                 190

Thr Val Ala Gly Arg Ala Asp Asp Phe Asp Gly Leu Leu His Tyr Gly
        195                 200                 205

Tyr Arg Gln Gly His Glu Thr Glu Ser Asn Gly Gly His Gly Gly Thr
    210                 215                 220

Gly Leu Ser Arg Ser Glu Ala Asn Pro Glu Asp Ala Asp Ser Tyr Ser
225                 230                 235                 240

Leu Leu Gly Lys Leu Gly Trp Asn Tyr Ala Glu Gly Ser Arg Phe Gly
                245                 250                 255

Leu Val Phe Glu Lys Tyr Lys Ser Asp Val Asp Thr Asp Gln Lys Ser
            260                 265                 270

Ala Tyr Gly Gly Pro Tyr Asp Lys Gly Lys Pro Ala Ile Pro Pro Ser
        275                 280                 285

Met Leu Pro Gly Gly Met Tyr Gln Trp Arg Lys Gly Asn Asp Thr Leu
    290                 295                 300

Thr Arg Glu Arg Tyr Gly Leu Glu His His Phe Leu Leu Asp Ser Gln
305                 310                 315                 320

Val Ala Asp Arg Ile Gln Trp Ser Leu Asn Tyr Gln Leu Ala Lys Thr
                325                 330                 335

Asp Gln Ala Thr Arg Glu Phe Tyr Tyr Pro Ile Thr Arg Lys Val Leu
            340                 345                 350

Arg Thr Arg Asp Thr Thr Tyr Lys Glu Arg Leu Trp Val Phe Asp Ser
        355                 360                 365

Gln Leu Asp Lys Ser Phe Ala Ile Gly Glu Thr Glu His Leu Leu Ser
    370                 375                 380

Tyr Gly Ile Asn Leu Lys His Gln Lys Val Thr Gly Met Arg Ser Gly
385                 390                 395                 400

Thr Gly Thr Asn Leu Asp Thr Gly Ala Asp Ser Pro Arg Asp Ala Leu
                405                 410                 415

Glu Arg Ser Ser Asp Phe Pro Asp Pro Thr Val Lys Thr Tyr Ala Leu
            420                 425                 430

Phe Ala Gln Asp Ser Ile Ser Trp Asn Asp Trp Thr Phe Thr Pro Gly
        435                 440                 445

Leu Arg Tyr Asp Tyr Thr Arg Met Glu Pro His Ile Thr Asp Glu Phe
    450                 455                 460

Leu Arg Thr Met Lys Gln Ser Gln Asn Thr Ala Val Asp Glu Ser Asp
465                 470                 475                 480

Lys Lys Trp His Arg Val Ser Pro Lys Phe Gly Val Thr Tyr Asp Phe
                485                 490                 495

Ala Gln His Tyr Thr Trp Tyr Gly Gln Tyr Ala Gln Gly Phe Arg Thr
            500                 505                 510

Pro Thr Ala Lys Ala Leu Tyr Gly Arg Phe Glu Asn Leu Gln Ala Gly
        515                 520                 525

Tyr His Ile Glu Pro Asn Pro Asn Leu Lys Pro Glu Lys Ser Gln Ser
    530                 535                 540

Phe Glu Thr Gly Leu Arg Gly Lys Phe Asp Glu Gly Ser Phe Gly Val
545                 550                 555                 560

Ala Val Phe Tyr Asn Lys Tyr Arg Asp Phe Ile Asp Glu Asp Ala Leu
                565                 570                 575

Asn Thr Asp Ser Thr Gly Gly Asn Gly Gln Thr Phe Gln Ser Asn Asn
            580                 585                 590

Ile Glu Arg Ala Val Ile Lys Gly Val Glu Leu Lys Gly Arg Leu Glu
        595                 600                 605

Leu Gly Ala Phe Gly Ala Pro Gln Gly Leu Tyr Thr Gln Gly Ser Val
    610                 615                 620

Ala Tyr Ala Tyr Gly Arg Asn Lys Asp Asn Gly Glu Pro Ile Asn Ser
625                 630                 635                 640

Val Asn Pro Leu Thr Gly Val Phe Gly Leu Gly Tyr Asp Glu Ala Asp
                645                 650                 655

Gly Asn Tyr Gly Gly Leu Leu Ser Trp Thr Leu Val Lys Arg Lys Asp
            660                 665                 670

Arg Val Asp Asp Ser Thr Phe His Thr Pro Asp Gly Thr Ala Ser Gln
        675                 680                 685

Phe Lys Thr Pro Gly Phe Gly Val Leu Asp Leu Ser Ala Tyr Tyr Arg
    690                 695                 700

Leu Ser Lys Asp Leu Thr Leu Asn Ala Gly Leu Tyr Asn Leu Thr Asp
705                 710                 715                 720

Lys Lys Tyr Trp Leu Trp Asp Asp Val Arg Gly Tyr Asp Ser Val Gly
                725                 730                 735

Glu Ala Ser Ala Leu Ala Pro Ala Asn Ile Asp Arg Leu Ser Gln Pro
            740                 745                 750

Gly Arg Asn Phe Ala Val Asn Leu Val Trp Asp Ile
        755                 760

<210>  4
<211>  584
<212>  PRT
<213>  Pseudomonas aeruginosa

<400>  4

Tyr Glu Ser Ala Ser His Ser Trp Leu Thr Ser Ala Thr Val Ala Gly
1               5                   10                  15

Arg Ala Asp Asp Phe Asp Gly Leu Leu His Tyr Gly Tyr Arg Gln Gly
            20                  25                  30

His Glu Thr Glu Ser Asn Gly Gly His Gly Gly Thr Gly Leu Ser Arg
        35                  40                  45

Ser Glu Ala Asn Pro Glu Asp Ala Asp Ser Tyr Ser Leu Leu Gly Lys
    50                  55                  60

Leu Gly Trp Asn Tyr Ala Glu Gly Ser Arg Phe Gly Leu Val Phe Glu
65                  70                  75                  80

Lys Tyr Lys Ser Asp Val Asp Thr Asp Gln Lys Ser Ala Tyr Gly Gly
                85                  90                  95

Pro Tyr Asp Lys Gly Lys Pro Ala Ile Pro Pro Ser Met Leu Pro Gly
            100                 105                 110

Gly Met Tyr Gln Trp Arg Lys Gly Asn Asp Thr Leu Thr Arg Glu Arg
        115                 120                 125

Tyr Gly Leu Glu His His Phe Leu Leu Asp Ser Gln Val Ala Asp Arg
    130                 135                 140

Ile Gln Trp Ser Leu Asn Tyr Gln Leu Ala Lys Thr Asp Gln Ala Thr
145                 150                 155                 160

Arg Glu Phe Tyr Tyr Pro Ile Thr Arg Lys Val Leu Arg Thr Arg Asp
                165                 170                 175

Thr Thr Tyr Lys Glu Arg Leu Trp Val Phe Asp Ser Gln Leu Asp Lys
            180                 185                 190

Ser Phe Ala Ile Gly Glu Thr Glu His Leu Leu Ser Tyr Gly Ile Asn
        195                 200                 205

Leu Lys His Gln Lys Val Thr Gly Met Arg Ser Gly Thr Gly Thr Asn
    210                 215                 220

Leu Asp Thr Gly Ala Asp Ser Pro Arg Asp Ala Leu Glu Arg Ser Ser
225                 230                 235                 240

Asp Phe Pro Asp Pro Thr Val Lys Thr Tyr Ala Leu Phe Ala Gln Asp
                245                 250                 255

Ser Ile Ser Trp Asn Asp Trp Thr Phe Thr Pro Gly Leu Arg Tyr Asp
            260                 265                 270

Tyr Thr Arg Met Glu Pro His Ile Thr Asp Glu Phe Leu Arg Thr Met
        275                 280                 285

Lys Gln Ser Gln Asn Thr Ala Val Asp Glu Ser Asp Lys Lys Trp His
    290                 295                 300

Arg Val Ser Pro Lys Phe Gly Val Thr Tyr Asp Phe Ala Gln His Tyr
305                 310                 315                 320

Thr Trp Tyr Gly Gln Tyr Ala Gln Gly Phe Arg Thr Pro Thr Ala Lys
                325                 330                 335

Ala Leu Tyr Gly Arg Phe Glu Asn Leu Gln Ala Gly Tyr His Ile Glu
            340                 345                 350

Pro Asn Pro Asn Leu Lys Pro Glu Lys Ser Gln Ser Phe Glu Thr Gly
        355                 360                 365

Leu Arg Gly Lys Phe Asp Glu Gly Ser Phe Gly Val Ala Val Phe Tyr
    370                 375                 380

Asn Lys Tyr Arg Asp Phe Ile Asp Glu Asp Ala Leu Asn Thr Asp Ser
385                 390                 395                 400

Thr Gly Gly Asn Gly Gln Thr Phe Gln Ser Asn Asn Ile Glu Arg Ala
                405                 410                 415

Val Ile Lys Gly Val Glu Leu Lys Gly Arg Leu Glu Leu Gly Ala Phe
            420                 425                 430

Gly Ala Pro Gln Gly Leu Tyr Thr Gln Gly Ser Val Ala Tyr Ala Tyr
        435                 440                 445

Gly Arg Asn Lys Asp Asn Gly Glu Pro Ile Asn Ser Val Asn Pro Leu
    450                 455                 460

Thr Gly Val Phe Gly Leu Gly Tyr Asp Glu Ala Asp Gly Asn Tyr Gly
465                 470                 475                 480

Gly Leu Leu Ser Trp Thr Leu Val Lys Arg Lys Asp Arg Val Asp Asp
                485                 490                 495

Ser Thr Phe His Thr Pro Asp Gly Thr Ala Ser Gln Phe Lys Thr Pro
            500                 505                 510

Gly Phe Gly Val Leu Asp Leu Ser Ala Tyr Tyr Arg Leu Ser Lys Asp
        515                 520                 525

Leu Thr Leu Asn Ala Gly Leu Tyr Asn Leu Thr Asp Lys Lys Tyr Trp
    530                 535                 540

Leu Trp Asp Asp Val Arg Gly Tyr Asp Ser Val Gly Glu Ala Ser Ala
545                 550                 555                 560

Leu Ala Pro Ala Asn Ile Asp Arg Leu Ser Gln Pro Gly Arg Asn Phe
                565                 570                 575

Ala Val Asn Leu Val Trp Asp Ile
            580

<210>  5
<211>  6
<212>  PRT
<213>  Pseudomonas aeruginosa

<400>  5

Glu Ser Ala Ser His Ser
1               5

<210>  6
<211>  28
<212>  PRT
<213>  Pseudomonas aeruginosa

<400>  6

Gly His Glu Thr Glu Ser Asn Gly Gly His Gly Gly Thr Gly Leu Ser
1               5                   10                  15

Arg Ser Glu Ala Asn Pro Glu Asp Ala Asp Ser Tyr

20                  25

<210>  7
<211>  40
<212>  PRT
<213>  Pseudomonas aeruginosa

<400>  7

Asp Val Asp Thr Asp Gln Lys Ser Ala Tyr Gly Gly Pro Tyr Asp Lys
1               5                   10                  15

Gly Lys Pro Ala Ile Pro Pro Ser Met Leu Pro Gly Gly Met Tyr Gln
            20                  25                  30

Trp Arg Lys Gly Asn Asp Thr Leu
        35                  40

<210>  8
<211>  27
<212>  PRT
<213>  Pseudomonas aeruginosa

<400>  8

Thr Asp Gln Ala Thr Arg Glu Phe Tyr Tyr Pro Ile Thr Arg Lys Val
1               5                   10                  15

Leu Arg Thr Arg Asp Thr Thr Tyr Lys Glu Arg
            20                  25

<210>  9
<211>  33
<212>  PRT
<213>  Pseudomonas aeruginosa

<400>  9

Gly Met Arg Ser Gly Thr Gly Thr Asn Leu Asp Thr Gly Ala Asp Ser
1               5                   10                  15

Pro Arg Asp Ala Leu Glu Arg Ser Ser Asp Phe Pro Asp Pro Thr Val
            20                  25                  30

Lys

<210>  10
<211>  27
<212>  PRT
<213>  Pseudomonas aeruginosa

<400>  10

Ile Thr Asp Glu Phe Leu Arg Thr Met Lys Gln Ser Gln Asn Thr Ala
1               5                   10                  15

Val Asp Glu Ser Asp Lys Lys Trp His Arg Val

20                  25

<210>  11
<211>  22
<212>  PRT
<213>  Pseudomonas aeruginosa

<400>  11

Tyr Gly Arg Phe Glu Asn Leu Gln Ala Gly Tyr His Ile Glu Pro Asn
1               5                   10                  15

Pro Asn Leu Lys Pro Glu
            20

<210>  12
<211>  23
<212>  PRT
<213>  Pseudomonas aeruginosa

<400>  12

Tyr Asn Lys Tyr Arg Asp Phe Ile Asp Glu Asp Ala Leu Asn Thr Asp
1               5                   10                  15

Ser Thr Gly Gly Asn Gly Gln
            20

<210>  13
<211>  16
<212>  PRT
<213>  Pseudomonas aeruginosa

<400>  13

Gly Arg Asn Lys Asp Asn Gly Glu Pro Ile Asn Ser Val Asn Pro Leu
1               5                   10                  15

<210>  14
<211>  16
<212>  PRT
<213>  Pseudomonas aeruginosa

<400>  14

Asp Ser Thr Phe His Thr Pro Asp Gly Thr Ala Ser Gln Phe Lys Thr
1               5                   10                  15

<210>  15
<211>  29
<212>  PRT
<213>  Pseudomonas aeruginosa

<400>  15

Asp Asp Val Arg Gly Tyr Asp Ser Val Gly Glu Ala Ser Ala Leu Ala
1               5                   10                  15

Pro Ala Asn Ile Asp Arg Leu Ser Gln Pro Gly Arg Asn
            20                  25

<210>  16
<211>  25
<212>  DNA
<213>  Artificial Sequence

<220>
<223>  primer

<400>  16
gggaaaggct gggagtgctg ctcat                                              25


<210>  17
<211>  25
<212>  DNA
<213>  Artificial Sequence

<220>
<223>  primer

<400>  17
gtggatcatg ggcgctccgt ttgcc                                              25


<210>  18
<211>  20
<212>  DNA
<213>  Artificial Sequence

<220>
<223>  primer

<400>  18
atgctgccgg gcggcatgta                                                    20


<210>  19
<211>  20
<212>  DNA
<213>  Artificial Sequence

<220>
<223>  primer

<400>  19
catggagccg cacatcaccg                                                    20


<210>  20
<211>  20
<212>  DNA
<213>  Artificial Sequence

<220>
<223>  primer

<400>  20
tacacccagg gcagcgtggc                                                    20


<210>  21
<211>  20
<212>  DNA
<213>  Artificial Sequence

<220>

<223>  primer

<400>  21
gacccaccgc aactacgtcg                                                    20


<210>  22
<211>  20
<212>  DNA
<213>  Artificial Sequence

<220>
<223>  primer

<400>  22
gccgtccttg atgatgtccg                                                    20


<210>  23
<211>  29
<212>  DNA
<213>  Artificial Sequence

<220>
<223>  primer

<400>  23
gactctcgag tacgagtcgg ccagccact                                          29


<210>  24
<211>  31
<212>  DNA
<213>  Artificial Sequence

<220>
<223>  primer

<400>  24
gactggatcc tcagatgtcc cagaccaggt t                                       31


<210>  25
<211>  25
<212>  PRT
<213>  Artificial Sequence

<220>
<223>  synthetic peptide

<400>  25

Cys Thr Glu Ser Asn Gly Gly His Gly Gly Thr Gly Leu Ser Arg Ser
1               5                   10                  15


Glu Ala Asn Pro Glu Asp Ala Asp Ser
            20                  25


<210>  26
<211>  15
<212>  PRT
<213>  Artificial Sequence

<220>
<223>  synthetic peptide

<400>  26

Cys Thr Asp Gln Lys Ser Ala Tyr Gly Gly Pro Tyr Asp Lys Gly
1               5                   10                  15


<210>  27
<211>  12
<212>  PRT
<213>  Artificial Sequence

<220>
<223>  synthetic peptide

<400>  27

Cys Gly Met Tyr Gln Trp Arg Lys Gly Asn Asp Thr
1               5                   10


<210>  28
<211>  21
<212>  PRT
<213>  Artificial Sequence

<220>
<223>  synthetic peptide

<400>  28

Cys Glu Phe Tyr Tyr Pro Ile Thr Arg Lys Val Leu Arg Thr Arg Asp
1               5                   10                  15


Thr Thr Tyr Lys Glu
            20


<210>  29
<211>  22
<212>  PRT
<213>  Artificial Sequence

<220>
<223>  synthetic peptide

<400>  29

Cys Thr Gly Ala Asp Ser Pro Arg Asp Ala Leu Glu Arg Ser Ser Asp
1               5                   10                  15


Phe Pro Asp Pro Thr Val
            20


<210>  30
<211>  15
<212>  PRT
<213>  Artificial Sequence

<220>
<223>  synthetic peptide

<400>  30

Cys Lys Gln Ser Gln Asn Thr Ala Val Asp Glu Ser Asp Lys Lys
1               5                   10                  15

<210>  31
<211>  15
<212>  PRT
<213>  Artificial Sequence

<220>
<223>  synthetic peptide

<400>  31

Cys Phe Glu Asn Leu Gln Ala Gly Tyr His Ile Glu Pro Asn Pro
1               5                   10                  15


<210>  32
<211>  10
<212>  PRT
<213>  Artificial Sequence

<220>
<223>  synthetic peptide

<400>  32

Cys Tyr Asn Lys Tyr Arg Asp Phe Ile Asp
1               5                   10


<210>  33
<211>  11
<212>  PRT
<213>  Artificial Sequence


<220>
<223>  synthetic peptide

<400>  33

Cys Leu Asn Thr Asp Ser Thr Gly Gly Asn Gly
1               5                   10


<210>  34
<211>  12
<212>  PRT
<213>  Artificial Sequence


<220>
<223>  synthetic peptide

<400>  34

Cys Gly Arg Asn Lys Asp Asn Gly Glu Pro Ile Asn
1               5                   10


<210>  35
<211>  14
<212>  PRT
<213>  Artificial Sequence


<220>
<223>  synthetic peptide

<400>  35

Cys Asp Ser Thr Phe His Thr Pro Asp Gly Thr Ala Ser Gln
1               5                   10


<210>  36
<211>  11
<212>  PRT
<213>  Artificial Sequence


<220>
<223>  synthetic peptide

<400>  36

Cys Asp Asp Val Arg Gly Tyr Asp Ser Val Gly
1               5                   10


<210>  37
<211>  11
<212>  PRT
<213>  Artificial Sequence


<220>
<223>  synthetic peptide

<400>  37

Cys Asn Ile Asp Arg Leu Ser Gln Pro Gly Arg
1               5                   10


<210>  38
<211>  10
<212>  PRT
<213>  Artificial Sequence


<220>
<223>  synthetic peptide

<400>  38

Cys Leu Arg Gly Lys Phe Asp Glu Gly Ser
1               5                   10


<210>  39
<211>  10
<212>  PRT
<213>  Artificial Sequence


<220>
<223>  synthetic peptide

<400>  39

Cys Tyr Asp Glu Ala Asp Gly Asn Tyr Gly
1               5                   10

## Description

### Technical Field

The present invention relates to: a protein antigen or a peptide antigen, which is derived from a Pseudomonas aeruginosa-outer membrane protein PA4710; and an antibody directed against the antigen. The present invention also relates to a vaccine composition comprising the antigen. The present invention further relates to a pharmaceutical composition, a diagnostic agent for a Pseudomonas aeruginosa infection, a therapeutic agent for a Pseudomonas aeruginosa infection, and a detection kit for Pseudomonas aeruginosa, which comprise the antibody.

### Background of the Invention

Pseudomonas aeruginosa is a gram-negative bacillus widely and generally distributed in natural environments such as soil and water, and causes refractory and serious fatal infections. A main target thereof is easily infective patients with attenuated biological defense mechanisms, including burned, organ-transplanted or cancer patients. Such patients are generally called compromised hosts. Pseudomonas aeruginosa is a major causative bacterium of hospital infections. Furthermore, the lung infections caused by this bacterium are fatal to cystic fibrosis patients. An antibacterial agent having an anti-Pseudomonas aeruginosa activity is mainly administered to these patients. However, sufficient therapeutic effects are not obtained in many cases due to the drug resistance of Pseudomonas aeruginosa. Alternatively, vaccines or antibodies directed against Pseudomonas aeruginosa have also been studied for years. However, the method directly using inactivated form of the bacteria has disadvantages that various types of vaccines and antibodies have to be individually prepared for the respective serotypes of Pseudomonas aeruginosa.

Under such a situation, the prevention or treatment of a Pseudomonas aeruginosa infection has been expected through active immunity or passive immunity acquired by using a protein derived from Pseudomonas aeruginosa, the protein having a common amino acid sequence among Pseudomonas aeruginosa strains. Known examples of a Pseudomonas aeruginosa-derived protein applied in the form of vaccines include: a recombinant protein in which portions of outer membrane proteins OprF and OprI are fused with each other (Japanese Unexamined Patent Application Publication No. Hei 8-245699: Document 1); a type IV pilin protein (WO 2004/099250: Document 2); and the like.

Moreover, reported as therapeutic antibodies directed against a protein derived from Pseudomonas aeruginosa are: an anti-type IV pilin antibody (Document 2); an anti-PA1706 (or PcrV) antibody (US 6309651: Document 3, US 6827935: Document 4); an anti-PA5158 antibody (WO 2007/049770: Document 5); and the like.

On the other hand, a bacteria-derived protein commonly possessed by clinical isolates of Pseudomonas aeruginosa which exhibit diverse serotypes is applicable as a "Pseudomonas aeruginosa common antigen" to the prevention, diagnosis or treatment of a Pseudomonas aeruginosa infection. Thus, such a protein has always been demanded.

Meanwhile, a PA4710 (also known as PhuR) protein encoded by a PA4710 (or phuR) gene (Genebank accession No. AF055999) is an outer membrane haemin receptor protein, constituting the haem uptake system of Pseudomonas aeruginosa belonging to the TonB-dependent receptor family (Microbiology, 2000, 146, 185-198: Document 6, Environmental Microbiology, 2003, 5, 1350-1369: Document 7). The TonB-dependent receptor contains an outer membrane β barrel and a plug domain, the outer membrane β barrel composed of 22 transmembrane β strand penetrating the outer membrane. The plug domain enters the β barrel from the periplasm side to plug the β barrel. When a ligand is bound, the TonB-dependent receptor changes its conformation, so that the ligand is incorporated into the periplasm. The TonB-dependent receptor requires energy to incorporate the ligand. This energy is supplied from an energy transducing complex through the interaction between the TonB protein and the TonB box extending in the amino terminal of the TonB-dependent receptor. The energy transducing complex consists of three proteins TonB, ExbB, and ExbD present in the inner membrane. Only D-Squared Biotechnologies, Inc. disclosed that a partial sequence of the PA4710 protein can be used as a vaccine component or that an antibody composition produced from the sequence can be used as an infection therapeutic agent or diagnostic agent. However, the disclosure is based solely on the homology of iron-uptake proteins among a broad range of bacterial species, not endorsed by conducted example (WO2002/083843: Document 8, WO 2003/006672: Document 9).

### Disclosure of the Invention

An object of the present invention is to provide: a protein antigen or a peptide antigen usable as a vaccine composition which has an ability to practically prevent or treat a Pseudomonas aeruginosa infection, and which can cope with the diversity of clinical isolates derived from patients infected with Pseudomonas aeruginosa; and an antibody directed against the antigen.

In order to achieve the above object, the present inventors have attempted to search a Pseudomonas aeruginosa-outer membrane protein for a novel and useful "Pseudomonas aeruginosa common antigen." As a result of various studies, the present inventors have found by GeneChip analysis that a gene encoding a PA4710 (also known as PhuR) protein present in the outer membrane of Pseudomonas aeruginosa is constantly expressed regardless of the presence or absence of human sera (Example 1). Moreover, by making gene analysis on 67 clinical isolates of Pseudomonas aeruginosa, the present inventors have successfully identified amino acid sequences-conserved regions of the PA4710 protein, and concurrently specified 11 extracellular regions within the amino acid sequences-conserved regions(Examples 2, 9).

Furthermore, the present inventors have found that an antiserum or antibody obtained by immunization with a PA4710 recombinant protein or peptide in the extracellular regions within the amino acid sequences-conserved regions binds to the PA4710 protein and binds also to the cell surface of Pseudomonas aeruginosa (Examples 7, 8). Moreover, the present inventors have confirmed that the antibody shows a potent protective effect against infections on Pseudomonas aeruginosa-infected model mice (Examples 10 to 12).

In other words, the present inventors have successfully narrowed down the immunodominant region of the PA4710 protein by making detailed analyses on the entire region of the PA4710 protein, and concurrently found that an antibody directed against the region shows a potent protective effect against infections on Pseudomonas aeruginosa-infected model mice, thereby leading to the attainment of the present invention.

More specifically, the present invention relates to the following inventions.
<1> A protein selected from the following (i), (ii), (iii), and (iv):
   (i) a protein comprising the amino acid sequence represented by SEQ ID NO: 4;
   (ii) a protein comprising an amino acid sequence in which one or more amino acids are deleted, substituted, inserted or added in the amino acid sequence represented by SEQ ID NO: 4, the protein being functionally equivalent to a protein consisting of the amino acid sequence represented by SEQ ID NO: 4;
   (iii) a protein encoded by a polynucleotide which hybridizes under a stringent condition to a polynucleotide encoding the amino acid sequence represented by SEQ ID NO: 4, the protein being functionally equivalent to the protein consisting of the amino acid sequence represented by SEQ ID NO: 4; and
   (iv) a protein comprising an amino acid sequence having 70% or more identity with the amino acid sequence represented by SEQ ID NO: 4, the protein being functionally equivalent to the protein consisting of amino acid sequence represented by SEQ ID NO: 4.
<2> A peptide consisting of an amino acid sequence included in an amino acid sequence selected from the group consisting of positions 181 to 198, 204 to 257, 259 to 311, 313 to 319, 321 to 436, 440 to 491, 493 to 600, and 602 to 764 in an amino acid sequence represented by SEQ ID NO: 3, wherein
   a peptide region exposed from a Pseudomonas aeruginosa surface is encoded.
<3> A peptide consisting of an amino acid sequence in which one or a plurality of amino acids are conservatively substituted in the amino acid sequence of the peptide according to <2>.
<4> A peptide consisting of an amino acid sequence represented by any one of SEQ ID NOS: 5 to 15.
<5> A peptide consisting of an amino acid sequence in which one or a plurality of amino acids are conservatively substituted in the amino acid sequence of the peptide according to <4>.
<6> A peptide consisting of at least 7 consecutive amino acids of the peptide according to <2>.
<7> A peptide consisting of at least 7 consecutive amino acids of the peptide according to <3>.
<8> A peptide consisting of at least 7 consecutive amino acids of the peptide according to <4>.
<9> A peptide consisting of at least 7 consecutive amino acids of the peptide according to <5>.
<10> A antibody or a functional fragment thereof, which is against a PA4710 protein or a portion thereof derived from Pseudomonas aeruginosa.
<11> The antibody or the functional fragment thereof according to <10>, wherein the portion of the PA4710 protein derived from Pseudomonas aeruginosa is a loop-containing cell surface region.
<12> An antibody or a functional fragment thereof, which is against the protein according to <1>.
<13> An antibody or a functional fragment thereof, which is against the peptide according to <2>.
<14> An antibody or a functional fragment thereof, which is against the peptide according to <3>.
<15> An antibody or a functional fragment thereof, which is against the peptide according to <4>.
<16> An antibody or a functional fragment thereof, which is against the peptide according to <5>.
<17> An antibody or a functional fragment thereof, which is against the peptide according to <6>.
<18> An antibody or a functional fragment thereof, which binds to a peptide consisting of any one of amino acid sequences of SEQ ID NOS: 5 to 15, but does not bind to a peptide consisting of any other amino acid sequences of SEQ ID NOS: 5 to 15.
<19> The antibody or the functional fragment thereof according to <10>, which binds to a surface of Pseudomonas aeruginosa.
<20> The antibody or the functional fragment thereof according to any one of <10> to <19>, wherein the antibody is a monoclonal antibody.
<21> The antibody or the functional fragment thereof according to <10>, which has an antibacterial activity in a patient infected with Pseudomonas aeruginosa.
<22> The antibody or the functional fragment thereof according to <21>, wherein the patient is a patient with a reduced neutrophil level.
<23> The antibody or the functional fragment thereof according to <21>, wherein the Pseudomonas aeruginosa is multidrug resistant Pseudomonas aeruginosa.
<24> The antibody or the functional fragment thereof according to any one of <21> to <23>, wherein the antibody is a monoclonal antibody.
<25> An antibody or a functional fragment thereof, which is produced by a hybridoma deposited under any one of accession numbers FERM BP-10970, FERM BP-10971, FERM BP-10972, FERM BP-10973, and FERM BP-10974.
<26> A monoclonal antibody or a functional fragment thereof, which reacts with an antigen identical to an antigen of a monoclonal antibody produced by a hybridoma deposited under any one of accession numbers FERM BP-10970, FERMBP-10971, FERM BP-10972, FERMBP-10973, and FERM BP-10974.
<27> A hybridoma producing the antibody according to <20>.
<28> A hybridoma producing the antibody according to < 24>.
<29> A hybridoma deposited under any of accession numbers FERM BP-10970, FERM BP-10971, FERM BP-10972, FERM BP-10973, and FERM BP-10974.
<30> An antigen composition comprising any one of a protein antigen and a peptide antigen which are capable of inducing production of an antibody directed against a PA4710 protein derived from Pseudomonas aeruginosa.
<31> An antigen composition comprising any one of the protein according to <1> and the peptide according to any one of <2> to <9>.
<32> A vaccine composition for use in prevention or treatment of a disease associated with Pseudomonas aeruginosa, the vaccine composition comprising the antigen composition according to any one of <30> and <31>, and optionally comprising at least one pharmaceutically acceptable carrier, diluent and/or adjuvant.
<33> The vaccine composition according to <32>, wherein the disease associated with Pseudomonas aeruginosa is a systemic infectious disease caused by a Pseudomonas aeruginosa infection
<34> The vaccine composition according to <33>, wherein the Pseudomonas aeruginosa infection is a multidrug resistant Pseudomonas aeruginosa infection.
<35> A pharmaceutical composition for use in prevention or treatment of a disease associated with Pseudomonas aeruginosa, the pharmaceutical composition comprising the antibody or the functional fragment thereof according to any one of <10> to <19>, <21> to <23>, <25>, and <26> and optionally comprising at least one pharmaceutically acceptable carrier and/or diluent.
<36> The pharmaceutical composition according to <35>, wherein the disease associated with Pseudomonas aeruginosa is a systemic infectious disease caused by a Pseudomonas aeruginosa infection.
<37> The pharmaceutical composition according to <36>, wherein the Pseudomonas aeruginosa infection is a multidrug resistant Pseudomonas aeruginosa infection.
<38> A diagnostic agent for a Pseudomonas aeruginosa infection, comprising the antibody or the functional fragment thereof according to any one of <10> to <19>, <25>, and <26>.
<39> A detection kit for Pseudomonas aeruginosa, comprising the antibody or the functional fragment thereof according to any one of <10> to <19>, <25>, and <26>.

### Detailed Description of the Preferred Embodiments

### [PA4710 Protein]

A PA4710 protein is an outer membrane protein derived from Pseudomonas aeruginosa. The amino acid sequence of the protein is described in SEQ ID NO: 3, and the base sequence of a polynucleotide encoding the protein is described in SEQ ID NO: 1.

In this context, on the basis of information obtained from structure analysis information about an Escherichia coli FhuA protein (Cell, 1998, 95, 771-778, and Science, 1998, 282, 2215-2220), structure analysis information about an Escherichia coli FepA protein (Nat. Struct. Biol., 1999, 6, 56-63), structure analysis information about an Escherichia coli FecA protein (Science, 2002, 295, 1715-1719, J. Mol. Biol., 2003, 332, 353-368), structure analysis information about a Pseudomonas aeruginosa FpvA protein (J. Mol. Biol., 2005, 347, 121-134) and secondary structure prediction information about the PA4710 protein, the following estimations have been made. Specifically, a base sequence (SEQ ID NO: 2) from positions 541 to 2295 in 2295 bases of an amino acid coding region within the base sequence represented by SEQ ID NO: 1 encodes a protein portion of an outer membrane β barrel of the PA4710 protein. This region is composed of 22 transmembrane antiparallel β strands penetrating the outer membrane. Among 21 loops connecting the strands to each other, 11 loops are exposed from the cell surface. Hereinafter, this region is referred to as a "loop-containing cell surface region".

The loop-containing cell surface region of the PA4710 protein is a protein selected from the following (i), (ii), (iii), and (iv) :
(i) a protein comprising the amino acid sequence represented by SEQ ID NO: 4;
(ii) a protein comprising an amino acid sequence in which one or more amino acids are deleted, substituted, inserted or added in the amino acid sequence represented by SEQ ID NO: 4, the protein being functionally equivalent to a protein consisting of the amino acid sequence represented by SEQ ID NO: 4;
(iii) a protein encoded by a polynucleotide which hybridizes under a stringent condition to a polynucleotide encoding the amino acid sequence represented by SEQ ID NO: 4, the protein being functionally equivalent to the protein consisting of the amino acid sequence represented by SEQ ID NO: 4; and
(iv) a protein comprising an amino acid sequence having 70% or more identity with the amino acid sequence represented by SEQ ID NO: 4, the protein being functionally equivalent to the protein consisting of amino acid sequence represented by SEQ ID NO: 4.

In the present description, the expression "amino acid sequence in which one or more amino acids are deleted, substituted, inserted or added in the amino acid sequence" means that modification has been carried out by well-known methods such as site-directed mutagenesis, or by mutation (for example, substitution) of multiple amino acids to an extent comparable to those naturally occurring. The number of amino acids to be modified is preferably 1 to 50, more preferably 1 to 30, further preferably 1 to 10, still further preferably 1 to 5, and most preferably 1 to 2.

A preferable example of the modified amino acid sequence of the PA4710 protein can be an amino acid sequence having conservative substitutions of one or a plurality (preferably, 1 to several; for example, 1, 2, 3, or 4) of amino acids.

In the present description, the term "conservative substitution" means that at least one amino acid residue is substituted with another chemically similar amino acid residue. Examples thereof include a case of substituting a certain hydrophobic residue with another hydrophobic residue, and a case of substituting a certain polar residue with another polar residue having the same electric charge. For each type of amino acids, functionally similar amino acids which can be substituted as described above are publically known in this technical field. Specifically, examples of nonpolar (hydrophobic) amino acids include alanine, valine isoleucine, leucine, proline, tryptophan, phenylalanine, and methionine. Examples of polar (neutral) amino acids include glycine, serine, threonine, tyrosine, glutamine, asparagine, and cysteine. Examples of positively charged (basic) amino acids include arginine, histidine, and lysine. Furthermore, examples of negatively charged (acidic) amino acids include aspartic acid and glutamic acid.

In the present description, the term "stringent condition" means that a membrane washing procedure after hybridization is carried out at a high temperature in a solution having a low salt concentration, specifically, washing conditions at, for example, 0.5×SSC concentration (1×SSC: 15 mM trisodium citrate and 150 mM sodium chloride) at 60°C for 15 minutes, and preferably, washing conditions at 0.5×SSC concentration in a 0.1% SDS solution at 60°C for 15 minutes.

The hybridization can be carried out according to a known method. Meanwhile, in a case of using a commercially-available library, the hybridization can be carried out according to a method described in the attached instruction.

In the present description, the term "identity" of base sequences or of amino acid sequences is used to mean the degree of coincidence between compared sequences of base or amino acid residues constituting each sequence. Any numerical value of such "identity" indicated in the present description may be a numerical value calculated using a homology search program known to those skilled in the art. Such a numerical value can easily be calculated using a default (initially set) parameter in FASTA or BLAST, for example.

The amino acid sequence having 70% or more identity with the amino acid sequence represented by SEQ ID NO: 4 can be an amino acid sequence having preferably 80% or more, more preferably 85% or more, further preferably 90% or more, still further preferably 95% or more, particularly preferably 98% or more, and most preferably 99% or more identity therewith.

In the present invention, if the amino acid sequence represented by SEQ ID NO: 4 is given, a nucleotide sequence encoding it can easily be determined. Thus, various nucleotide sequences encoding the amino acid sequence represented by SEQ ID NO: 4 can be selected. Accordingly, a polynucleotide encoding the protein comprising the amino acid sequence represented by SEQ ID NO: 4 means not only a part or whole of the DNA sequence represented by SEQ ID NO: 2, but also DNA sequences encoding the same amino acid, and including degenerate codons. In the present invention, the polynucleotide further includes an RNA sequence corresponding to these.

A preferred example of the polynucleotide encoding the protein comprising the amino acid sequence represented by SEQ ID NO: 4 includes a polynucleotide comprising the base sequence represented by SEQ ID NO: 2.

In the present description, whether or not a certain protein is functionally equivalent to the protein consisting of the amino acid sequence represented by SEQ ID NO: 4 can be determined by evaluating a biological phenomenon or function associated with the expression of the protein consisting of the amino acid sequence represented by SEQ ID NO: 4. For example, it can be determined by allowing the certain protein to express by genetic recombination technique and then evaluating whether or not an antibody directed against the PA4710 protein can be prepared.

Since the protein of the present invention exists on the cell surface of Pseudomonas aeruginosa, the protein can be used as an antigen (protein antigen) for preparing an antibody directed against Pseudomonas aeruginosa.

By analysis of numerous clinical isolates, the present inventors have successfully specified amino acid sequences-conserved regions within the PA4710 protein (SEQ ID NO: 3). The specified regions (having at least 5 amino acids) are as follows:
positions 181 to 198, 204 to 257, 259 to 311, 313 to 319, 321 to 436, 440 to 491, 493 to 600, and 602 to 764 in the amino acid sequence represented by SEQ ID NO: 3.

A peptide of the present invention is preferably a peptide which is included in these amino acid sequences-conserved regions, and concurrently which is in peptide regions exposed from the Pseudomonas aeruginosa surface (hereinafter, referred to as "extracellular regions"). This is for producing an antibody for use in a medicament and a diagnostic agent for a Pseudomonas aeruginosa infection. Such a peptide serves as a common antigen. The extracellular regions can be specified by analysis of the structural feature of the PA4710 protein, verification by experiment in which an antibody is bound to the Pseudomonas aeruginosa surface, or the like (see Examples 2, 9). The present inventors have specified 11 peptide regions (SEQ ID NOS: 5 to 15) as the extracellular region. SEQ ID NOS: 5 to 15 are preferable forms of the peptide of the present invention.

It is confirmed that antibody targeting peptides consisting of amino acid sequences represented by SEQ ID NOS: 11, 12, and 14 among the peptides of SEQ ID NOS: 5 to 15 of the present invention have an antibacterial activity against a Pseudomonas aeruginosa infection (Examples 11, 12). Accordingly, the peptides consisting of the amino acid sequence represented by SEQ ID NOS: 11, 12, and 14 are particularly favorable peptides each serving as an antigen for preparing an antibody for use in a medicament against a Pseudomonas aeruginosa infection.

The peptide of the present invention, which is used for preparing an antibody, is not necessarily the entire peptide of the extracellular regions. As long as the preparation of the antibody is possible, the chain length of the amino acids is not limited. The chain length is preferably 7 or more amino acids (for example, 8 or more amino acids, 10 or more amino acids, and 12 or more amino acids).

When used as the common antigen, the peptide of the present invention is preferably a peptide which is included in the amino acid sequences-conserved regions described above. Meanwhile, when the peptide of the present invention is used for other purposes (for example, when a particular strain of Pseudomonas aeruginosa is targeted, and so on), it is conceivable that a region including a mutation is targeted. In this manner, the peptide of the present invention includes one having one or several amino acids mutated. Such a mutation can be a conservative substitution.

The peptide of the present invention may have a blocking group added to the N-terminal or C-terminal thereof, for example, so as to prevent aggregation attributed to electric charges. Acetylation and amidation are often used for the N-terminal and the C-terminal, respectively, but not limited to these. For example, modification may be used for the peptide of the present invention by adding a cysteine residue thereto, so as to enhance binding with a spacer.

DMS (Dimethyl Suberimidate), DMA (Dimethyl adipimidate), Sulfo-SMCC (Sulfosuccinimidyl-4-[N-maleimidomethyl]cyclohexane-1-carboxylate), Sulfo-MBS (m-Maleimidobenzoyl-N-hydroxysulfosuccinimide ester), or the like is generally used as the spacer, but not limited to these. A compound functioning as the spacer is sufficient therefor.

For the peptide of the present invention, carrier proteins such as bovine serum albumin (BSA), ovalbumin (OVA), human serum albumin (HSA), or hemocyanin derived from grand keyhole limpet (KLH Keyhole limpet hemocyanin) can be used as carriers, but not limited to these. The peptide may be incorporated into a terminal or inside of another protein to prepare a fused protein, and the protein thus fused can be used as an antigen for preparing an antibody.

### [Antigen Composition]

The protein of the present invention or the peptide of the present invention can be used as a protein antigen or a peptide antigen. Thus, according to the present invention, provided is an antigen composition comprising any one of the protein antigen and the peptide antigen which are capable of inducing production of an antibody directed against an outer membrane PA4710 protein derived from Pseudomonas aeruginosa.

In this context, the protein antigen or the peptide antigen can preferably be used by purifying the protein of the present invention or the peptide of the present invention according to a method well known to those skilled in the art.

In the present description, the "antigen composition" may be a composition consisting of only the protein antigen or the peptide antigen as a constituent thereof, or a composition additionally comprising other components.

According to the present invention, provided is an antigen composition comprising any one of a protein antigen and a peptide antigen which are capable of inducing production of an antibody directed against a PA4710 protein derived from Pseudomonas aeruginosa.

### [Antibody]

An antibody of the present invention can recognize a Pseudomonas aeruginosa-outer membrane PA4710 protein or a portion thereof, and bind to Pseudomonas aeruginosa.

According to the present invention, provided is an antibody or a functional fragment thereof of the present invention, wherein the portion of the PA4710 protein derived from Pseudomonas aeruginosa is a loop-containing cell surface region.

In this context, the loop-containing cell surface region of the PA4710 protein is a protein selected from the following (i), (ii), (iii), and (iv):
(i) a protein comprising the amino acid sequence represented by SEQ ID NO: 4;
(ii) a protein comprising an amino acid sequence in which one or more amino acids are deleted, substituted, inserted or added in the amino acid sequence represented by SEQ ID NO: 4, the protein being functionally equivalent to a protein consisting of the amino acid sequence represented by SEQ ID NO: 4;
(iii) a protein encoded by a polynucleotide which hybridizes under a stringent condition to a polynucleotide encoding the amino acid sequence represented by SEQ ID NO: 4, the protein being functionally equivalent to the protein consisting of the amino acid sequence represented by SEQ ID NO: 4; and
(iv) a protein comprising an amino acid sequence having 70% or more identity with the amino acid sequence represented by SEQ ID NO: 4, the protein being functionally equivalent to the protein consisting of amino acid sequence represented by SEQ ID NO: 4.

According to the present invention, provided is an antibody or a functional fragment thereof of the present invention, wherein the portion of the PA4710 protein derived from Pseudomonas aeruginosa is: (i) a peptide being the amino acid sequence-conserved region (positions 181 to 198, 204 to 257, 259 to 311, 313 to 319, 321 to 436, 440 to 491, 493 to 600, or 602 to 764 in the amino acid sequence represented by SEQ ID NO: 3) and being the extracellular region; (ii) a peptide consisting of an amino acid sequence in which one or a plurality of amino acids are conservatively substituted in the amino acid sequence of the peptide according to (i); or (iii) a peptide consisting of at least 7 consecutive amino acids of the peptide according to (i) or (ii).

According to the present invention, provided is an antibody or a functional fragment thereof of the present invention, wherein the portion of the PA4710 protein derived from Pseudomonas aeruginosa is: (i) a peptide being an extracellular loop region represented by any one of SEQ ID NOS: 5 to 15; (ii) a peptide consisting of an amino acid sequence in which one or a plurality of amino acids are conservatively substituted in the amino acid sequence of the peptide according to (i); or (iii) a peptide consisting of at least 7 consecutive amino acids of the peptide according to (i)or (ii).

According to the present invention, provided is an antibody which binds only to a particular extracellular loop region among the extracellular loop regions represented by the amino acid sequences of SEQ ID NOS: 5 to 15 within the PA4710 protein derived from Pseudomonas aeruginosa, but does not bind to the other extracellular loop regions.

According to the present invention, provided is an antibody capable of binding to Pseudomonas aeruginosa, the antibody being characterized by being produced by the immune system of an animal itself in response to the antigen composition of the present invention.

The antibody of the present invention can be used in treatment or diagnosis of a Pseudomonas aeruginosa infection, or as a reagent for research. In the embodiment of the antibody of the present invention applied to a Pseudomonas aeruginosa infection, provided is an antibody or a functional fragment thereof having an antibacterial activity in a patient infected with Pseudomonas aeruginosa. In this embodiment, a particularly preferable antibody is an antibody directed against the peptide consisting of the amino acid sequence represented by any one of SEQ ID NOS: 11, 12, and 14. When the antibody is activated against a region of the peptide consisting of the amino acid sequence represented by any one of SEQ ID NOS: 11, 12, and 14 within the PA4710 protein (SEQ ID NO: 3), the antibacterial activity against Pseudomonas aeruginosa can be exhibited. Exemplified as a specific antibody showing such an antibacterial activity are antibodies produced by hybridomas deposited under accession numbers of, for example, FERM BP-10972, FERM BP-10973, and FERM BP-10974. Once a peptide region preferable as a target of such an antibody is specified in order that the antibody exhibits the antibacterial activity against Pseudomonas aeruginosa, those skilled in the art are able to prepare various antibodies showing the same activity as described above while targeting the peptide region. The present inventors have found out such target peptide regions, and the present invention includes various antibodies that bind to such peptide regions.

The patient infected with Pseudomonas aeruginosa can be, for example, a patient with a reduced neutrophil level due to administrations of various drugs, radiotherapy, or the like. The antibody of the present invention is advantageous in that the antibody is capable of exhibiting the effect on such a patient who is thus likely to develop serious infection. Meanwhile, Pseudomonas aeruginosa with which the patient is infected can be multidrug resistant Pseudomonas aeruginosa. The antibody of the present invention is advantageous in that the antibody shows the effectiveness on a patient infected with multidrug resistant Pseudomonas aeruginosa who cannot be treated with generally-used antibiotics.

According to the present invention, provided is an antibody or functional fragment thereof, which is produced by a hybridoma deposited under any one of accession numbers of FERM BP-10970, FERM BP-10971, FERM BP-10972, FERM BP-10973, and FERM BP-10974. Furthermore, provided is an antibody characterized by being a monoclonal antibody which reacts with an antigen identical to an antigen of a monoclonal antibody produced by these hybridomas.

The antibody of the present invention is preferably obtained by administering a purified antigen composition to an experimental animal in such an amount that the antibody can be induced, the purified antigen composition comprising the protein antigen or the peptide antigen of the present invention. A pure antibody can be prepared by collecting blood from the heart or artery, separating antisera therefrom, and purifying the obtained antisera.

The antibody of the present invention includes: a polyclonal antibody or a monoclonal antibody, which is obtained by immunizing a mammal such as a mouse with an antigen, the PA4710 protein or peptide serving as the antigen (including the monoclonal antibody produced by the hybridoma that produces the monoclonal antibody of the present invention); a chimeric antibody and a humanized antibody, which are prepared by using genetic recombination technique; and a human antibody prepared by using a human antibody-producing transgenic animal or the like. When the antibody of the present invention is administered as a medicament to a human, the human antibody is desirable in terms of reducing side effects.

The "human antibody" is an antibody having all of regions derived from a human. The human antibody of the present invention can be prepared using a method well known to those skilled in the art (can be referred to, for example, Intern. Rev. Immunol, 1995, 13, 65-93, J. Mol. Biol, 1991, 222, 581-597, Japanese Unexamined Patent Application Publication No. Hei 10-146194, Japanese Unexamined Patent Application Publication No. Hei 10-155492, Japanese Patent No. 2938569, Japanese Unexamined Patent Application Publication No. Hei 11-206387, Japanese Patent Translation Publication No. Hei 8-509612, Japanese Patent Translation Publication No. Hei 11-505107, and the like).

The "humanized antibody" is an antibody prepared by transplanting only the gene sequence of the antigen-binding site (CDR; complementarity determining region) of a mouse antibody into a human antibody gene (CDR grafting). The humanized antibody of the present invention can be prepared using a method well known to those skilled in the art (can be referred to, for example, EP 239400, WO 90/07861, and the like).

The "chimeric antibody" is an antibody prepared by ligating the variable region of an antibody of a certain species to the constant region of an antibody of a different species. Specifically, a mouse is immunized with an antigen in order to prepare a monoclonal antibody, and a variable region (V region) that binds to the antigen is cut out of the gene of the mouse monoclonal antibody. The obtained V region is then allowed to bind to a constant region (C region) gene derived from human bone marrow. In this manner, the chimeric antibody can be prepared. The chimeric antibody of the present invention can be prepared using a method well known to those skilled in the art (can be referred to, for example, Japanese Patent Application Publication No. Hei 8-280387, US Patent No. 4816397, US Patent No. 4816567, US Patent No. 5807715, and the like).

The monoclonal antibody of the present invention can be prepared using a method well known to those skilled in the art (can be referred to, for example, Antibodies A LABORATORY MANUAL, Ed Harlow and David Lane, Cold Spring Harbor Laboratory 1988; Experimental Manual for Monoclonal Antibody (1987) Kodansha, edited by Sakuji Toyama et al.; Monoclonal Antibody-Hybridoma and ELISA (1987) Kodansha, edited by Tatsuo Iwasaki, et al; and the like).

The polyclonal antibody of the present invention can be prepared using a method well known to those skilled in the art.

The term "functional fragment" of the present invention means a part (a partial fragment thereof) of an antibody, which specifically recognizes the protein of the present invention. Specific examples thereof include Fab, Fab', F(ab')₂, variable region fragment (Fv), disulfide-bonded Fv, a single chain antibody (scFv), and polymers thereof.

Moreover, according to the present invention, provided is a hybridoma producing the antibody of the present invention. As the preferable embodiment of the hybridoma of the present invention, provided are hybridomas deposited at the National Institute of Advanced Industrial Science and Technology, International Patent Organism Depositary (Central 6, 1-1-1, Higashi, Tsukuba, Ibaraki, postal code 305-8566, Japan) on May 28, 2008, under the accession numbers of FERM BP-10970, FERM BP-10971, FERMBP-10972, FERMBP-10973, and FERM BP-10974. The corresponding original deposits are as follows:

A hybridoma (4710-B-1) under the accession number of FERM P-20723, a hybridoma (4710-L3A-1) under FERM P-20724, and a hybridoma (4710-L7-1) under FERM P-20725, deposited at the National Institute of Advanced Industrial Science and Technology, International Patent Organism Depositary (Central 6, 1-1-1, Higashi, Tsukuba, Ibaraki, postal code 305-8566, Japan) on November 25, 2005; and a hybridoma (4710-L8B-1) under the accession numbers of FERM P-21205 and a hybridoma (4710-L10-1) under FERM P-21206 deposited at the same institute on February 8, 2007.

### [Vaccine Composition]

The antigen composition of the present invention can be used as a vaccine. Thus, according to the present invention, provided is a vaccine composition comprising an antigen composition capable of inducing production of an antibody directed against the outer membrane PA4710 protein derived from Pseudomonas aeruginosa.

According to the present invention, a vaccine composition for use in prevention or treatment of a disease associated with Pseudomonas aeruginosa can be prepared, the vaccine composition comprising the antigen composition according to the present invention, and optionally comprising at least one pharmaceutically acceptable carrier, diluent, and/or adjuvant.

The carrier used in the vaccine composition of the present invention is selected on the basis of the mode and route of administration, and actual standard drug formulation. The carrier may be carrier proteins (for example, bovine serum albumin (BSA), ovalbumin (OVA), human serum albumin (HSA), hemocyanin derived from grand keyhole limpet (KLH: Keyhole limpet hemocyanin), and the like), solubilizers (for example, ethanol, polysorbate, Cremophor EL (registered trademark), and the like), isotonic agents, preservatives, antioxidants, excipients (for example, lactose, starch, crystalline cellulose, mannitol, maltose, calcium hydrogen phosphaste, light anhydrous silicic acid, calcium carbonate, and the like), binders (for example, starch, polyvinypyrrolidone, hydroxypropylcellulose, ethylcellulose, carboxymethylcellulose, gum arabic, and the like), lubricants (for example, magnesium stearate, talc, hydrogenated oil, and the like), stabilizers (for example, lactose, mannitol, maltose, polysorbate, macrogol, polyoxyethylene hydrogenated castor oil, and the like), and the like. If necessary, glycerin, dimethylacetamide, 70% sodium lactate, a surfactant, a basic substance (for example, sodium hydroxide, ethylenediamine, ethanolamine, sodium bicarbonate, arginine, meglumine, trisaminomethane, or the like), or the like may be added.

As a specific example of the carrier protein, the peptide of the present invention can be coupled to a known KLH solution (manufactured by Calbiotec Inc., 125 mg is dissolved per ml of a 50% glycerol solution), so as to enhance the antigenicity of the vaccine composition of the present invention.

The diluent used in the vaccine composition of the present invention is selected on the basis of the mode and route of administration, and actual standard drug formulation. Examples of the diluents include water or a saline, a phosphate-buffered saline, and a bicarbonate solution.

The adjuvant used in the vaccine composition of the present invention is selected on the basis of the mode and route of administration, and actual standard drug formulation. Examples of the adjuvant include cholera toxin, Escherichia coli heat-labile enterotoxin (LT), liposome, an immunostimulating complex (ISCOM: immunostimulating complex), and the like.

An administration may differ depending on the age, weight, sex, and general health state of an administration target at a risk of a Pseudomonas aeruginosa infection. The administration can be carried out by any administration route of oral administration and parenteral administration (for example, intravenous administration, intraarterial administration, and local administration). However, parenteral administration is preferable. The dosage form for oral administration and parenteral administration and the preparation method thereof are well known to those skilled in the art. The dosage form can be prepared according to a conventional process, for example, by mixing the antigen composition of the present invention with the aforementioned pharmaceutically acceptable carrier or the like. Examples of the dosage form for oral administration include solid and liquid dosage forms, and specifically a solution, a tablet, a granule, a powder, and a capsule. Examples of the dosage form for parenteral administration include a solution, a suspension, an ointment, a cream, a suppository, an ophthalmic agent, nasal drops, and ear drops. In the case of oral administration, a flavoring agent and a coloring agent can also be added.

If the sustained release of the present preparation is desired, a biodegradable polymer (for example, poly-D,L-lactide-co-glycolide, polyglycolide, or the like) can be added as a bulk matrix (can be referred to, for example, US Patent No. 5,417,986, US Patent No. 4,675,381, and US Patent No. 4,450,150).

Appropriate pharmaceutical carrier, diluents, and the like, as well as pharmaceutically necessities for their use are described in Remington's Pharmaceutical Sciences.

The dose of the vaccine composition of the present invention is determined by the present inventors depending on, for example, the type of vaccine antigen, whether or not the adjuvant is administered in combination with the present antigen, the type of adjuvant coadministered therewith, the mode and frequency of administration, and a desired effect (for example, a preventive or therapeutic effect). Generally, the dose of the vaccine composition of the present invention is 1 µg to 100 mg per administration for one adult. When the adjuvant is administered in combination with the present vaccine, the dose is generally 1 ng to 1 mg per administration for one adult. In accordance with the decision made by the present inventors, the administration is repeated when necessary. For example, following the initial administration, 3 booster administrations can be carried out per week. Alternatively, using the same formulations, a booster injection can be carried out on the 8th to 12th week after the first immunization and a second booster injection can be carried out on the 16th to 20th week thereafter.

### [Use of Antibody and Pharmaceutical Composition]

### Disease Associated with Pseudomonas aeruginosa

Pseudomonas aeruginosa is a pathogen of opportunistic infections which cause fatal consequences with reductions in the resistance of hosts. Moreover, since being resistant to antibiotics, Pseudomonas aeruginosa is a major causative bacterium of hospital infections. As shown in Examples described later, it has been confirmed that the antibody of the present invention actually has a protective effect against infections on a Pseudomonas aeruginosa infection-susceptible murine model with macrophage functions reduced by mucin administration (Example 10), and that the antibody of the present invention actually has a protective effect against infections on a Pseudomonas aeruginosa infection-susceptible murine model with a neutrophil level reduced by cyclophosphamide monohydrate administration (Example 11). Furthermore, it has been confirmed that the antibody of the present invention actually has a protective effect against infections on a multidrug resistant Pseudomonas aeruginosa infection-susceptible murine model (Example 12). Thus, activating the antibody of the present invention against the Pseudomonas aeruginosa-PA4710 protein (particularly, the extracellular region) can prevent or treat the disease associated with Pseudomonas aeruginosa. The antibody of the present invention can be used against Pseudomonas aeruginosa of various natures and also for Pseudomonas aeruginosa-infected patients with various symptoms. Within the extracellular region of the PA4710 protein, a region of the peptide consisting of the amino acid sequence represented by any one of SEQ ID NOS: 11, 12, and 14 is a region particularly favorably targeted by the antibody for such medical purposes. Specific examples of the antibody showing the antibacterial activity in Pseudomonas aeruginosa-infected patients include the antibodies produced by the hybridomas deposited under the accession numbers of FERM BP-10972, FERM BP-10973, and FERM BP-10974. The antibody directed against the region of the peptide consisting of the amino acid sequence represented by SEQ ID NO: 14 within the extracellular region of the PA4710 protein (for example, the antibody produced by the hybridoma deposited under the accession number of FERM BP-10974) can be favorably used for prevention or treatment of a multidrug resistant Pseudomonas aeruginosa infection which is difficult to treat (Example 12).

Examples of the disease associated with Pseudomonas aeruginosa include systemic infectious diseases, caused by a Pseudomonas aeruginosa infection including a multidrug resistant Pseudomonas aeruginosa infection, for example, septicemia, meningitis, and endocarditis. Other examples thereof include: otitis media and sinusitis in the otolaryngologic field; pneumonia, chronic respiratory tract infection, and catheter infection in the pulmonary field; postoperative peritonitis and postoperative infection in a biliary duct or the like in the surgical field; abscess of eyelid, absecess of nasolacrimal duct, conjunctivitis, corneal ulcer, corneal abscess, panophthalmitis, and orbital infection in the ophthalmological field; and urinary tract infections including complicated urinary tract infection, catheter infection, and abscess around the anus in the urologic field. Besides, the examples include burns (including a serious burn and a burn of the respiratory tract), decubital infection, and cystic fibrosis.

According to the present invention, provided is a prevention method or treatment method of the disease associated with Pseudomonas aeruginosa, the method comprising a step of administering a preventively or therapeutically effective amount of the antibody of the present invention to mammals including a human.

### Diagnostic Agent for Pseudomonas aeruginosa Infection

As shown in Examples described later, it has been confirmed that the antibody of the present invention binds to the extracellular region of the PA4710 protein exposed from the cell surface of Pseudomonas aeruginosa (Example 8, 9). This result suggests that the antibody of the present invention be capable of detecting the presence of Pseudomonas aeruginosa. Thus, the antibody of the present invention can be used as a diagnostic agent for a Pseudomonas aeruginosa infection. The antibody that binds to the region of the peptide consisting of the amino acid sequence represented by any one of SEQ ID NOS: 11, 12, and 14 within the extracellular region of the PA4710 protein is a particularly favorable antibody in such diagnosis.

According to the present invention, provided is a diagnosis method for a Pseudomonas aeruginosa infection using the antibody of the present invention. The diagnosis method of the present invention can be carried out by collecting a biological sample such as sputum, a lung lavage fluid, pus, a tear, blood, or urine from mammals including a human at a risk of a Pseudomonas aeruginosa infection, subsequently bringing the collected sample into contact with the antibody of the present invention, and determining whether or not an antigen-antibody reaction occurs.

### Diagnostic Agent Kit for Pseudomonas aeruginosa Infection

According to the present invention, provided is a kit for detecting the presence of Pseudomonas aeruginosa, the kit comprising at least the antibody of the present invention.

The antibody of the present invention may be one which is labeled. This kit for detection detects the presence of Pseudomonas aeruginosa by detecting the antigen-antibody reaction.

Thus, the detection kit of the present invention can further include various reagents for carrying out the antigen-antibody reaction, a secondary antibody used, for example, in an ELISA method, a chromogenic reagent, a buffer, instructions, and/or an instrument, etc. if desired.

### Pharmaceutical Composition

A pharmaceutical composition or an agent of the present invention may be used in the form of a composition which uses the antibody of the present invention as an active ingredient, and preferably which contains a purified antibody composition and another component, for example, a saline, an aqueous glucose solution or a phosphate buffer.

The pharmaceutical composition of the present invention may be formulated in a liquid or freeze-dried form as necessary, and may optionally comprise a pharmaceutically acceptable carrier, for example, a stabilizer, a preservative, and an isotonic agent.

Examples of the pharmaceutically acceptable carrier can include: mannitol, lactose, saccharose, and human albumin for a freeze-dried preparation; and saline, water for injection, a phosphate buffer, and aluminium hydroxide for a liquid preparation. However, the examples are not limited to these.

An administration may differ depending on the age, weight, sex, and general health state of an administration target. The administration can be carried out by any administration route of oral administration and parenteral administration (for example, intravenous administration, intraarterial administration, and local administration). However, parenteral administration is preferable.

The dose of the pharmaceutical composition varies depending on the age, weight, sex, and general health state of a patient, the severity of a Pseudomonas aeruginosa infection and components of an antibody composition to be administered. The dose of the antibody composition of the present invention is generally 0.1 to 1000 mg, and preferably 1 to 100 mg, per kg body weight per day for an adult through intravenous injection.

The pharmaceutical composition of the present invention is preferably administered in advance to a patient at a risk of a Pseudomonas aeruginosa infection.

When the pharmaceutical composition is prepared as a diagnostic agent, the diagnostic agent can be obtained in any dosage form by adopting any means suitable for its purpose. For example, ascites, a culture solution containing an antibody of interest, or a purified antibody is measured for the antibody titer and appropriately diluted with PBS (phosphate buffer containing a saline) or the like; thereafter, a preservative such as 0.1% sodium azide is added thereto. Alternatively, the antibody of the present invention adsorbed to latex or the like is determined for the antibody titer and appropriately diluted, and a preservative is added thereto for use. The antibody of the present invention bound to latex particles as described above is one of preferable dosage forms as a diagnostic agent. As the latex in this case, appropriate resin materials, for example, latex such as polystyrene, polyvinyl toluene, or polybutadiene, are suitable.

### [Examples]

Hereinbelow, the present invention will be described in line with Examples to promote the understanding of the present invention. However, the present invention is not limited to these Examples.

### [Example 1] : GeneChip^{R} Analysis

GeneChip^{R} expression analysis system (manufactured by Affymetrix Inc., GeneChip^{R} P. aeruginosa genome array) was used as an approach for searching a human sera-added medium for genes that are expressed therein. Shake culture was carried out using a Pseudomonas aeruginosa PAO1 strain under three different culture conditions, i.e., in Luria-Bertani (LB) media (manufactured by NACALAI TESQUE, INC.) to which 0%, 20%, and 50% human sera were respectively added (the final compositions of the LB media were equal to one another) at 37°C until the absorbance at 595 nm reached 1.0. Using RNeasy Protect Bacteria Mini kit (Manufactured by QIAGEN GmbH), total RNA was extracted according to the method in documents attached thereto, and quantified using 2100 Bioanalyzer (manufactured by Agilent Technologies, Inc.). Then, the experiment was carried out according to the method in documents attached to GeneChip^{R}. The gene expression data was analyzed using Microarray Suite 5.0 (manufactured by Affymetrix Inc.), and signal and detection were calculated. At this time, correction was carried out, such that the average value of signals from all probe sets was 1000. Two independent experiments were carried out.

As a result, under any of the culture conditions regardless of the presence or absence of the added sera, a PA4761 protein (DnaK or HSP70), which is a house keeping protein, was determined to be "Present" that indicates a transcription product has been detected. It was thus shown that the gene was expressed. Moreover, a PA2018 protein (MexY) (J. Bacteriology, 2005, 187, 5341-5346), which is a transmembrane protein penetrating the inner membrane that associates with a PA5158 protein (OpmG) and a PA2019 protein (MexX) so as to constitute a drug efflux pump, and which is induced by ribosome inhibitors such as tetracycline or aminoglycoside antibiotics, was determined to be "Absent" under the conditions at this time that those drugs were not present. It was thus shown that the genes thereof were not expressed. By contrast, a PA4710 gene was determined to be "Absent" under the condition that no sera was added. It was thus shown that the gene was not expressed. Meanwhile, the PA4710 gene was determined to be "Present" under the conditions that the sera were added.

Therefore, it was suggested that the PA4710 gene was certainly expressed, and that there is a possibility that its gene product, PA4710 protein, is constantly present on the bacterial surface. This suggested that the Pseudomonas aeruginosa-PA4710 protein be useful as a vaccine component.

### [Example 2]: Analysis of PA4710 Gene in Clinical Isolates

Bacterial strains used and subjected to tests were 67 Pseudomonas aeruginosa strains (stored in Yokohama Research Lab., Meiji Seika Kaisha, Ltd.) isolated from various clinical materials in clinical facilities all over Japan. These strains were derived from blood, urine, sputum, pus, pharyngeal mucus, and the like. Their serotypes include groups A, B, E, G, I, M, etc. based on serological classification according to the decision made by the serotyping committee sponsored by Japan Pseudomonas aeruginosa Society (1975).

### (1) Preparation of Genomic DNA

Each of 67 clinical isolates of Pseudomonas aeruginosa was cultured overnight at 37°C in a Muller-Hinton medium (manufactured by Becton, Dickinson and Company), and collected by low-speed centrifugation. Using DNeasy Tissue kit (Manufactured by QIAGEN GmbH), genomic DNA was prepared from the obtained bacterial cells according to the method in documents attached thereto.

### (2) Amplification of DNA Fragment by PCR Method

Using the prepared genomic DNA as a template, a region including a PA4710 gene was amplified by PCR. Specifically, a primer set (SEQ ID NO: 16 and SEQ ID NO: 17) for specifically amplifying the PA4710 gene was designed based on the genomic sequence of a Pseudomonas aeruginosa PAO1 strain (NCBI database accession number: NC_002516). Using GeneAmp PCR System 9700 (manufactured by Applied BioSystems Inc.), PCR was carried out with Takara ExTaq (manufactured by Takara Bio Inc.) according to the attached instruction. The DNA fragment thus amplified by PCR was confirmed by agarose gel electrophoresis to have the size of interest (2635 base pairs).

### (3) Analysis of Polynucleotide Sequence Using DNA Sequencer

The PCR product was purified using MultiScreen PCR plate (manufactured by Millipore Corporation), and then subjected to a sequencing reaction. Primers (SEQ ID NO: 18 to SEQ ID NO: 22) capable of sequencing each PCR product were designed based on the genomic sequence of the PAO1 strain (NC_002516). BigDye Terminator vl. 1 Cycle Sequencing kit (manufactured by Applied BioSystems Inc.) was used in the sequencing reaction. The sequencing reaction was carried out using GeneAmp PCR System 9700 (manufactured by Applied BioSystems Inc.) according to the attached instruction. The sequencing reaction product was purified using MultiScreen-HV plate (manufactured by Millipore Corporation) filled with Sephadex G-50 Fine DNA Grade (manufactured by Amersham Biosciences AB) which had been swollen with water in advance. Then, the polynucleotide sequence was analyzed using Applied BioSystems 3730 DNA Analyzer (manufactured by Applied BioSystems Inc.).

The polynucleotide sequences of the clinical isolates revealed by the analysis were converted into polypeptide sequences, and these polypeptide sequences were compared with those from the PAO1 strain. As a result, 17 mutations were observed in the full-length sequence of the PA4710 protein (Table 1).

Furthermore, extracellular regions (SEQ ID NO: 5 to SEQ ID NO: 15) were found out within peptide regions (amino acid sequences-conserved regions) not including the 17 mutations by structure analysis based on information obtained from structure analysis information about an Escherichia coli FhuA protein (Cell, 1998, 95, 771-778, and Science, 1998, 282, 2215-2220), structure analysis information about an Escherichia coli FepA protein (Nat. Struct. Biol., 1999, 6, 56-63), structure analysis information about an Escherichia coli FecA protein (Science, 2002, 295, 1715-1719, and J. Mol. Biol., 2003, 332, 353-368), structure analysis information about a Pseudomonas aeruginosa FpvA protein (J. Mol. Biol., 2005, 347, 121-134) and secondary structure prediction information about the PA4710 protein. These peptides in the extracellular regions of the Pseudomonas aeruginosa-PA4710 protein are useful as a "Pseudomonas aeruginosa common antigen."

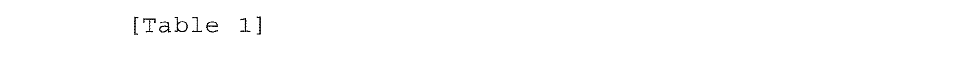

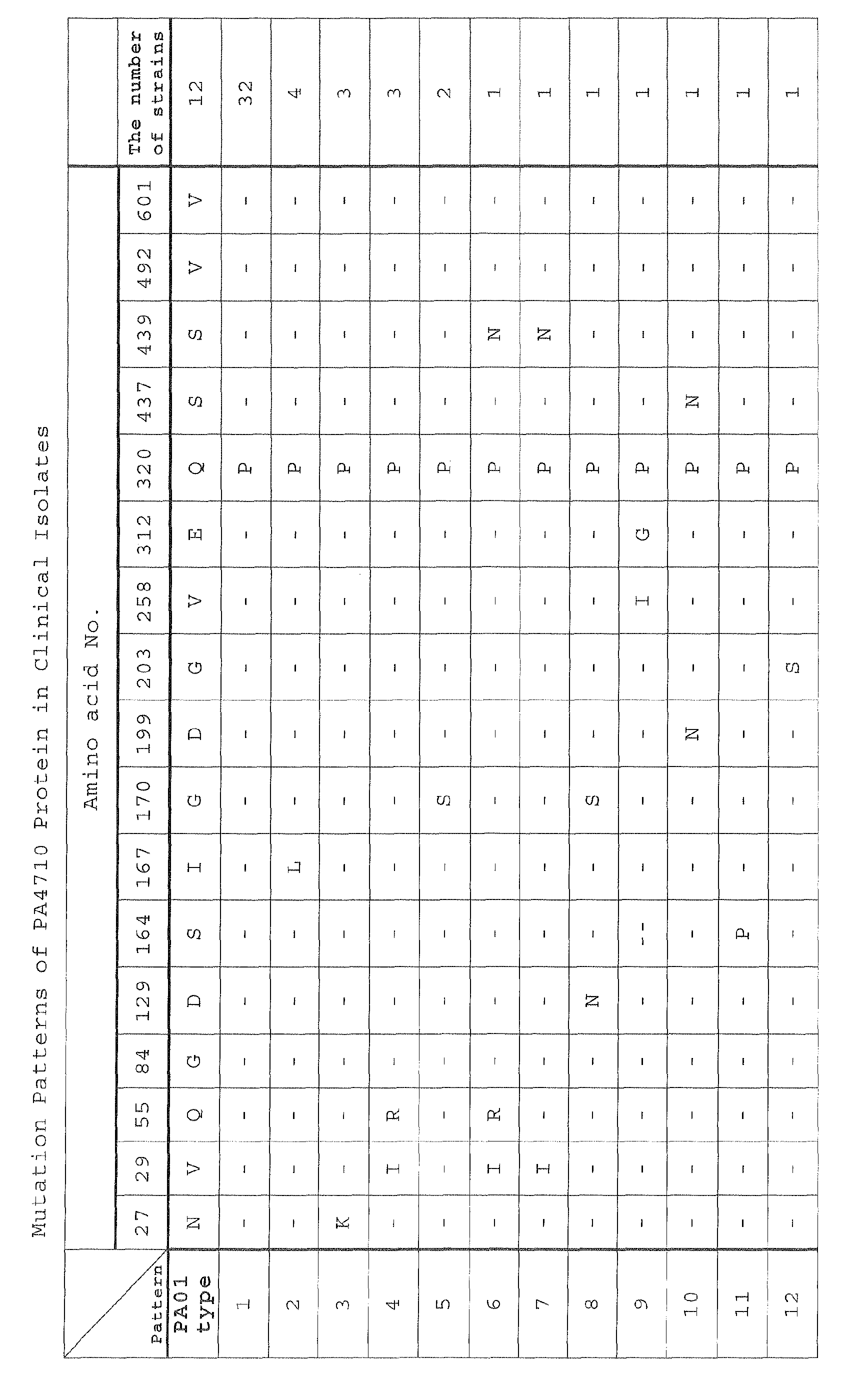

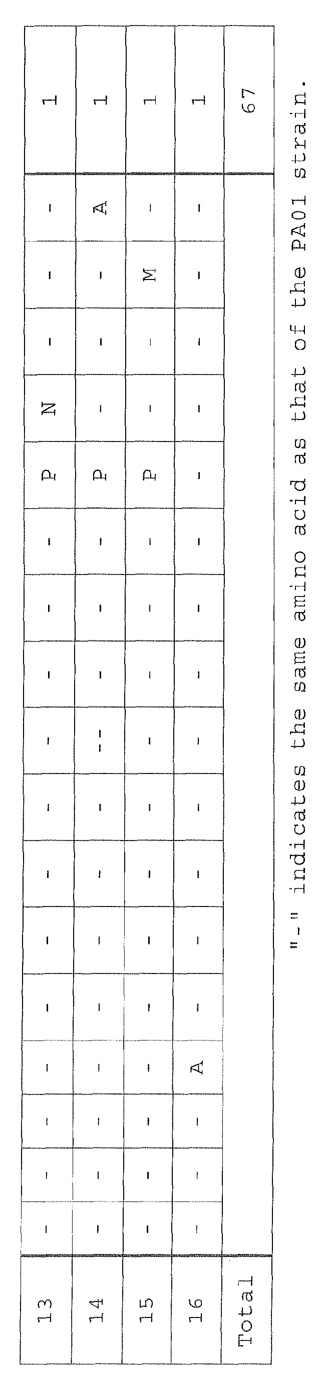

### [Example 3] : Cloning of PA4710 Gene DNA Fragment

A DNA fragment (SEQ ID NO: 2) from positions 541 to 2295 in 2295 bases of an amino acid coding region within a Pseudomonas aeruginosa-PA4710 gene (SEQ ID NO: 1) was incorporated into a cell-free protein expression vector pIVEX2.4d (Roche Diagnostics K. K.) and an Escherichia coli expression vector pET15b (Novagen Inc.) by the following method.

On the basis of a signal sequence as well as the structure analysis information about the Escherichia coli FhuA protein belonging to the same TonB-dependent receptor family as the PA4710 protein (Cell, 1998, 95, 771-778, and Science, 1998, 282, 2215-2220), the structure analysis information about the Escherichia coli FepA protein (Nat. Struct. Biol., 1999, 6, 56-63), the structure analysis information about the Escherichia coli FecA protein (Science, 2002, 295, 1715-1719, and J. Mol. Biol., 2003, 332, 353-368), the structure analysis information about the Pseudomonas aeruginosa FpvA protein (J. Mol. Biol., 2005, 347, 121-134) and the secondary structure prediction information about the PA4710 protein, it was estimated that a base sequence from positions 1 to 540 in the amino acid coding region encodes a portion not exposed from the cell surface. Thus, this base sequence was excluded from the cloning target.

The DNA fragment to be cloned was amplified from the genomic DNA of the Pseudomonas aeruginosa PAO1 strain by PCR (DNA Thermal Cycler 480; manufactured by Perkin-Elmer Inc.). Pyrobest (manufactured by TAKARA SHUZO CO., LTD.) was used as a DNA polymerase. Five percent of dimethyl sulfoxide was added to a reaction solution. Primers (SEQ ID NO: 23 and SEQ ID NO: 24) containing bases used for adding restriction sites XhoI (CTCGAG) and BamHI (GGATCC) were used as PCR primers.

The temperature conditions for PCR involved heating at 94°C for 2 minutes, and subsequent 30 cycles consisting of: 94°C for 30 seconds; 60°C for 1 minute; and 72°C for 2 minutes. The PCR product was purified using GenElute PCR DNA Purification Kit (manufactured by Sigma-Aldrich Co.), and then digested with XhoI (manufactured by New England Biolabs Inc.) and BamHI (manufactured by Toyobo Co., Ltd.). pIVEX2.4d was digested with XhoI and BamHI. These DNA fragments were electrophoresed on agarose gel, and extracted and purified using QIAquick Gel Extraction Kit (manufactured by Qiagen GmbH). The PCR product thus digested with XhoI-BamHI and pIVEX2.4d were ligated using T4 DNA ligase (manufactured by Invitrogen Corporation), and transformed into an Escherichia coli DH5α strain (Competent High DH5α, manufactured by Toyobo Co., Ltd.). A pIVEX2.4d plasmid (pIVEX-PA4710-1) having the PA4710 gene fragment incorporated therein was purified using QIAprep Spin Miniprep Kit (manufactured by Qiagen GmbH). Then, a cycle sequencing reaction was carried out using BigDye Terminator vl.1 Cycle Sequencing Kit (manufactured by Applied BioSystems Inc.), and the base sequence of the inserted portion was confirmed using 3730 DNA Analyzer ( manufactured by Applied BioSystems Inc./HITACHI Ltd.).

Next, pET15b was digested with XhoI and BamHI, and ligated to the XhoI-BamHI insertion fragment of pIVEX-PA4710-1, thereby transforming Escherichia coli. Thus, a pET15b plasmid (pET-PA4710-4) having the PA4710 gene fragment incorporated therein was obtained.

### [Example 4] : Expression and Purification of PA4710 Recombinant Protein

A Cell-free system and an Escherichia coli expression system were used for expression of a recombinant protein.

As the cell-free system, RTS 500 ProteoMaster E. coli HY Kit (manufactured by Roche Diagnostic K. K.) for carrying out transcription and translation with a T7 RNA polymerase and an Escherichia coli lysate was used. The cell-free system protein expression vector pIVEX-PA4710-1 is a plasmid encoding a His-tag(6 consecutive histidines)-PA4710 fusion protein downstream of a T7 promoter (see Example 3). A cell-free system reaction solution was prepared according to an instruction manual. A reaction was carried out at 30°C for 20 hours by addition of 10 µg of pIVEX-PA4710-1, and the produced insoluble protein was collected by centrifugation.

As the Escherichia coli expression system, an expression system (manufactured by Novagen Inc.) comprising an Escherichia coli BL21 (DE3) strain having a T7 RNA polymerase gene incorporated therein and a pET vector having a T7 promoter was used. An Escherichia coli expression vector pET-PA4710-4 is a plasmid encoding a His-tag-PA4710 fusion protein downstream of the T7 promoter (see Example 3). The BL21 (DE3) strain was treated with calcium chloride (see Molecular Cloning 2nd ed., Sambrook et al. (1989)) and transformed with the pET-PA4710-4. The transformant was cultured overnight in an LB medium containing 50 µg/ml ampicillin, and diluted 200-fold in a fresh medium and suspended. After 4 hours of culturing at 37°C, IPTG was added at a final concentration of 0.5 mM, and the culturing was continued for additional 3 hours. The cells were collected by centrifugation, and frozen at -20°C. The cells were dissolved in a protein extraction reagent (BugBuster Protein Extraction Reagent; manufactured by Novagen Inc.), and inclusion bodies were collected according to the attached instruction. In this procedure, ultrasonication was additionally carried out, and lysozyme (egg-white lysozyme, manufactured by Seikagaku Corporation) was used at a final concentration of 200 µg/ml.

Ni chelate chromatography utilizing the His-tag was used for protein purification. The insoluble protein expressed in the cell-free system or the Escherichia coli expression system was solubilized with a dissolution buffer (Dulbecco's phosphate-buffered saline (PBS) to which 8 M urea, 5 mM imidazole, 200 mM NaCl and 0.05% NP-40 had been added). The dissolved protein was bound to Ni-NTA Agarose (manufactured by Qiagen GmbH), and washed with 40 volumes of a dissolution buffer. The protein was further washed with 40 volumes of a wash buffer (a dissolution buffer from which NP-40 was excluded). Then, the His-tag-attached protein was eluted with an elution buffer (PBS to which 8 M urea, 300 mM imidazole, and 200 mM NaCl had been added), and collected.

As a result, 1.7 mg of the protein was finally obtained from 1 ml of the reaction solution in the cell-free system, and 7.2 mg of the protein was finally obtained from 100 ml of the culture in the Escherichia coli expression system.

### [Example 5]: Immunization with Antigen and Preparation of Sera

Inactivated bacteria for use were obtained as follows. A Pseudomonas aeruginosa PA103 strain (ATCC29260) was cultured overnight at 37°C on a Muller-Hinton agar medium. Several colonies thereof were suspended in an LB medium, then shake-cultured overnight at 37°C, and washed with PBS and resuspended. Subsequently, inactivation treatment was carried out for 24 hours or longer by addition of 1% formalin. For use in immunization, the PA4710 recombinant protein was dissolved in an 8 M urea solution, so as to be 100 µg/ml.

Within the amino acid sequences (SEQ ID NO: 5 to SEQ ID NO: 15) in the extracellular regions found within amino acid sequences-conserved regions of the PA4710 protein, the peptides containing SEQ ID NO: 6 to SEQ ID NO: 15 were synthesized by a solid-phase synthesis method using Fmoc. The peptides of SEQ ID NO: 25 to SEQ ID NO: 37 were synthesized by adding a cysteine residue to the amino terminal of the amino acid sequences of SEQ ID NO: 6 to SEQ ID NO: 15 and amidating the carboxyl terminal..

In a synthetic peptide 4710L2 (SEQ ID NO: 25) including the amino acid sequence of 4710Loop2 (SEQ ID NO: 6), [M+1] m/z 2446.199 (calculated value: m/z 2447.451) was observed by mass spectrometry. By HPLC analysis, a peak thereof was given at a retention time of 10.265 minutes with an area ratio of 88.29%.

In a synthetic peptide 4710L3A (SEQ ID NO: 26) including the amino acid sequence of 4710Loop3 (SEQ ID NO: 7), [M+1] m/z 1587.823 (calculated value: m/z 1588.713) was observed by mass spectrometry. By HPLC analysis, a peak thereof was given at a retention time of 12.205 minutes with an area ratio of 59.02%.

In a synthetic peptide 4710L3B (SEQ ID NO: 27) including the amino acid sequence of 4710Loop3 (SEQ ID NO: 7), [M+1] m/z 1461.256 (calculated value: m/z 1457.65) was observed by mass spectrometry. By HPLC analysis, a peak thereof was given at a retention time of 15.568 minutes with an area ratio of 73.71%.

In a synthetic peptide 4710L4 (SEQ ID NO: 28) including the amino acid sequence of 4710Loop4 (SEQ ID NO: 8), [M+1] m/z 2683.446 (calculated value: m/z 2682.107) was observed by mass spectrometry. By HPLC analysis, a peak thereof was given at a retention time of 13.117 minutes with an area ratio of 73.64%.

In a synthetic peptide 4710L5 (SEQ ID NO: 29) including the amino acid sequence of 4710Loop5 (SEQ ID NO: 9), [M+1] m/z 2335.175 (calculated value: m/z 2335.495) was observed by mass spectrometry. By HPLC analysis, a peak thereof was given at a retention time of 12.629 minutes with an area ratio of 83.12%.

In a synthetic peptide 4710L6 (SEQ ID NO: 30) including the amino acid sequence of 4710Loop6 (SEQ ID NO: 10), [M+1] m/z 1681.271 (calculated value: m/z 1679.824) was observed by mass spectrometry. By HPLC analysis, a peak thereof was given at a retention time of 9.656 minutes with an area ratio of 73.45%.

In a synthetic peptide 4710L7 (SEQ ID NO: 31) including the amino acid sequence of 4710Loop7 (SEQ ID NO: 11), [M+1] m/z 1731.422 (calculated value: m/z 1730.92) was observed by mass spectrometry. By HPLC analysis, a peak thereof was given at a retention time of 14.669 minutes with an area ratio of 56.67%.

In a synthetic peptide 4710L8A (SEQ ID NO: 32) including the amino acid sequence of 4710Loop8 (SEQ ID NO: 12, [M+1] m/z 1337.108 (calculated value: m/z 1335.501) was observed by mass spectrometry. By HPLC analysis, a peak thereof was given at a retention time of 14.011 minutes with an area ratio of 81.31%.

In a synthetic peptide 4710L8B (SEQ ID NO: 33) including the amino acid sequence of 4710Loop8 (SEQ ID NO: 12), [M+1] m/z 1037.734 (calculated value: m/z 1037.066) was observed by mass spectrometry. By HPLC analysis, a peak thereof was given at a retention time of 10.083 minutes with an area ratio of 70.18%.

In a synthetic peptide 4710L9 (SEQ ID NO: 34) including the amino acid sequence of 4710Loop9 (SEQ ID NO: 13), [M+1] m/z 1316.107 (calculated value: m/z 1315.426) was observed by mass spectrometry. By HPLC analysis, a peak thereof was given at a retention time of 11.011 minutes with an area ratio of 70.61%.

In a synthetic peptide 4710L10 (SEQ ID NO: 35) including the amino acid sequence of 4710Loop10 (SEQ ID NO: 14), [M+1] m/z 1467.748 (calculated value: m/z 1465.514) was observed by mass spectrometry. By HPLC analysis, a peak thereof was given at a retention time of 13.276 minutes with an area ratio of 65.81%.

In a synthetic peptide 4710L11A (SEQ ID NO: 36) including the amino acid sequence of 4710Loop11 (SEQ ID NO: 15), [M+1] m/z 1186.740 (calculated value: m/z 1184.244) was observed by mass spectrometry. By HPLC analysis, a peak thereof was given at a retention time of 12.609 minutes with an area ratio of 55.14%.

In a synthetic peptide 4710L11B (SEQ ID NO: 37) including the amino acid sequence of 4710Loop11 (SEQ ID NO: 15), [M+1] m/z 1258.403 (calculated value: m/z 1257.434) was observed by mass spectrometry. By HPLC analysis, a peak thereof was given at a retention time of 14.268 minutes with an area ratio of 61.47%.

For synthesis of peptides including amino acid sequences in the intracellular region, a solid-phase synthesis method with Fmoc was used. Synthesized were peptides each having the carboxyl terminal amidated, and each having a cysteine residue added to the amino terminal.

In a synthetic peptide 4710A (SEQ ID NO: 38) including the intracellular region-amino acid sequence existing between 4710Loop7 (SEQ ID NO: 11) and 4710Loop8 (SEQ ID NO: 12) in the extracellular region, [M+1] m/z 1110.919 (calculated value: m/z 1110.25) was observed by mass spectrometry. By HPLC analysis, a peak thereof was given at a retention time of 13.687 minutes with an area ratio of 71.88%.

In a synthetic peptide 4710C (SEQ ID NO: 39) including the intracellular region-amino acid sequence existing between 4710Loop9 (SEQ ID NO: 13) and 4710Loop10 (SEQ ID NO: 14) in the extracellular region, [M+1] m/z 1107.356 (calculated value: m/z 1105.097) was observed by mass spectrometry. By HPLC analysis, a peak thereof was given at a retention time of 10.992 minutes with an area ratio of 58.52%.

Furthermore, each of the aforementioned synthetic peptides was coupled to Keyhole limpet hemocyanin (KLH) with a spacer, so as to simultaneously prepare a conjugated peptide. Sulfo-SMCC (Sulfosuccinimidyl-4-[N-maleimidomethyl]cyclohexane-1-carboxylate: manufactured by Pierce Biotechnology Inc.) was used as the spacer. The peptide synthesis and the KLH conjugated peptide preparation were entrusted to hermo ELECTRON Corporation.

For use in immunization, the KLH conjugated peptide was dissolved in an 8 M urea solution so that the final concentration of each KLH conjugated peptide was 83.3 µg/ml. In an immunization method for animals, a male BN rat (purchased from Charles River Laboratories Japan, Inc.) or a female New Zealand white rabbit (purchased from Charles River Laboratories Japan, Inc.) was subcutaneously or intramuscularly administered with 6 shots in total in combination with a Freund complete adjuvant only in the first shot, and in combination with an incomplete adjuvant in the subsequent shots, at 2-week intervals. For the immunization, 20 µg of each of the formalin-inactivated bacteria and the PA4710 recombinant protein was administered per animal, and 41.7 µg of the KLH conjugated peptides was administered per animal. One week after the final immunization, whole blood was collected from the carotid artery or abdominal aorta. The blood was left at room temperature for one hour, and then centrifuged (1500G, 20 minutes), so as to obtain approximately 5 ml/rat and approximately 50 ml/rabbit of supernatants as antisera.

### [Example 6]: Purification of IgG Fraction from Antisera and Ascites

An IgG fraction from the rat antisera and ascites were purified according to the ammnoium sulfate precipitation method by McCauley R & Racker, E( Molecular and Cellular Biochemistry 1, 73-81 (1973) ).

In the ammnoium sulfate precipitation method, an ice-colded saturated ammonium sulfate solution (pH 8) was added to the antisera to prepare a 43 (v/v) % suspension. The obtained suspension was stirred at room temperature for 15 minutes. Precipirates were collected by centrifugation at 10,000 × g for 20 minutes, and dissolved in a 10 mM potassium phosphate buffer (pH 8) to which 10% glycerol had been added. Then, an ice-colded saturated ammonium sulfate solution (pH 8) was added to prepare a 50 (v/v) % solution, and again precipitates were deposited and washed twice. The precipitates were dissolved in a 10 mM pottasium phosphate buffer (pH 8) to which 10% glycerol had been added, and then was dialyzed against the buffer overnight. The dialysate was centrifuged, and then applied to anion-exchange chromatography (DEAE-Toyopearl 650M (manufactured by TOSOH CORPORATION)). The ultraviolet absorption at 280 nm was measured, and the flow-through fraction was collected as an IgG fraction. The final sample was concentrated using Amicon Ultra-15 (Millipore Corporation), and the buffer was finally exchanged with a PBS (-) solution. Of a protein, 54 mg was collected as an IgG fraction by purification from 5 ml of the PA4710 recombinant protein immunized-rat antisera. The purified IgG fraction thus obtained was designated as anti-PA4710 IgG. The protein was quantified according to DC Protein Assay (manufactured by Bio-Rad Laboratories, Inc.) based on the Lowry method, and the IgG purity was evaluated by SDS-PAGE.

Moreover, used as an alternative simple method was the method of Harlow & Lane(Antibodies, A Laboratory Manual, Cold Spring Harbor (1988), Chapter 8, 288-318) in which caprylic acid is used. The rat antisera or ascites obtained by proliferating a rat-mouse hybridoma in the mouse abdominal cavity were centrifuged at 10,000 × g for 20 minutes. An insoluble matter was removed therefrom, and a supernatant was obtained. Two volumes of 60 mM sodium acetate (pH 4.0) were added to the supernatant, and then the pH was adjusted to 4.8 with 1N hydrochloric acid. Of caprylic acid, 0.06 volumes relative to the ascites sample were gradually added at room temperature, and the mixture was stirred for 30 minutes, so as to produce an insoluble matter. Precipitates were removed by centrifugation at 13,000 × g for 10 minutes. The resulting solution was then passed through a 0.45 µm filter. The obtained sample was concentrated using Amicon Ultra-15 (manufactured by Millipore Corporation), and the resultant was finally exchanged with a PBS (-) solution, so as to obtain a final sample. Of proteins, 24 mg, 6.5 mg, 2.7 mg, 4.5 mg, 6.8 mg, and 5.2 mg were collected as IgG fractions by purification from 10 ml of the rat antisera or 11 mL, 9 mL, 11 mL, 40 mL, and 36 mL of the mouse ascites including rat MAb produced from hybridomas under the accession numbers FERM BP-10970, FERM BP-10971, FERM BP-10972, FERM BP-10973, and FERM BP-10974 at the National Institute of Advanced Industrial Science and Technology, International Patent Organism Depositary. The proteins were quantified according to DC Protein Assay (manufactured by Bio-Rad Laboratories, Inc.) based on the Lowry method, and the IgG purity was evaluated by SDS-PAGE.

### [Example 7]: ELISA Test

In order to detect by the ELISA method an antibody that binds to the PA4710 recombinant protein, the PA4710 recombinant protein was dissolved in PBS to which 8 M urea has been added. Of the protein, 0.5 µg was placed per well of a 96-well nickel plate (HIS-Select High Sensitivity (HS) Nickel Coated Plates, manufactured by Sigma-Aldrich Co.). The plate was left at room temperature for one hour, so as to cause the binding of the protein to the plate. The plate was washed with a wash buffer (PBS to which 0.05% Tween 20, 5mM imidazole, and 500 mM NaCl had been added), and blocked with a blocking buffer (a wash buffer to which 0.5% gelatin had been added). Then, a sample including the antibody obtained in Example 5 or 6 was placed in the well and allowed for reaction for 30 minutes. The plate was washed thereafter. A secondary antibody (peroxidase-labeled goat anti-rat IgG antibody, 10000 fold diluted, manufactured by Sigma-Aldrich Co.) was placed therein and allowed for reaction for 30 minutes, and the plate was washed thereafter. A chromogenic substrate (TMB Microwell Peroxidase Substrate System, manufactured by KPL Inc.) was added for reaction, and then the enzyme reaction was terminated with 1 M phosphoric acid. Then, the absorbance at 450 nm was measured.

As a result, the absorbance of the PA4710 recombinant protein immunized-rat sera (10,000 fold diluted) was 0.676, whereas the absorbance of negative control sera before immunization (10,000 fold diluted) was 0.058. This indicates that the antibody that binds to the PA4710 recombinant protein as an immunogen is contained in the PA4710 recombinant protein immunized-rat sera.

Meanwhile, in order to detect by the ELISA method an antibody that binds to each of the synthetic peptides (SEQ ID NOS: 25 to 39), the synthetic peptide was dissolved in a carbonate buffer (0.15% Na₂CO₃, 0.3% NaHCO₃), and 1 µg of the peptide was placed per well of a 96-well plate (Maxisorp, manufactured by Nunc). The plate was left at 4°C overnight, so as to cause the peptide to adsorb on the plate. The plate was washed with PBS, and blocked with PBS to which 0.5% bovine serum albumin had been added. Then, a sample including the antibody obtained in Example 5 or 6 was diluted, placed in the well, and allowed for reaction for 2 hours. The plate was thereafter washed with PBS containing 0.05% Tween 20. A secondary antibody was placed in the well and allowed for reaction for one hour, and the plate was thereafter washed with PBS containing 0.05% Tween 20. The coloring occurred as in the above case, and the absorbance was measured. The result will be shown in Example 9 described later.

### [Example 8]: Whole cell ELISA Test

For Whole cell ELISA, a bacterial solution of the PA103 strain cultured in an LB medium was dispensed into an ELISA plate (MaxiSorp Type, manufactured by Nunc), followed by immobilization at 4°C. Then, the plate was washed with a wash buffer (TBS containing 0.05% Tween 20), and blocked with a blocking buffer (TBS containing 2% bovine serum albumin). Then, the sera obtained in Example 5 or the purified IgG fraction, which had been diluted with PBS, were added thereto as a primary antibody sample and allowed for reaction at 37°C for one hour. After washing, a peroxidase-labeled goat anti-rat IgG antibody (5000 fold diluted, manufactured by Sigma-Aldrich Co.) was used as a secondary antibody, and a chromogenic substrate (TMB Microwell Peroxidase substrate System, manufactured by KPL Inc.) was added for a reaction, and then the enzyme reaction was terminated with 0.18 M sulfuric acid. Then, the absorbance at 450 nm was measured.

As a result, with regard to the PA4710 recombinant protein immunized-rat sera, the absorbance of negative control rat sera before immunization (100 fold diluted) was 0.142, whereas that of the PA4710 recombinant protein immunized-rat sera (100 fold diluted) was 0.462. This indicates that the antibody (IgG) which recognizes the extracellular region of the PA4710 protein exposed from the bacterial cell surface is contained in the PA4710 recombinant protein immunized-rat sera.

Meanwhile, with regard to the anti-PA4710 IgG that is the purified IgG fraction obtained from the PA4710 recombinant protein immunized-rat sera, the absorbance of a negative control IgG fraction (50 µg/well) purified from control rat sera obtained by administering only adjuvant was 0.116, whereas that of the anti-PA4710 IgG (50 µg/well) was 0.377. This indicates that the antibody (IgG) which recognizes the extracellular region of the PA4710 protein exposed from the cell surface is contained in the IgG fraction.

### [Example 9]: Preparation of Monoclonal Antibody (MAb)

One week after the final immunization with the PA4710 recombinant protein or the KLH conjugated peptide in Example 5, the spleen was aseptically extracted from a rat under anesthesia. The obtained spleen was washed with an RPMI-1640 medium (manufactured by Gibco Corp.). Then, the spleen was inserted between slide glasses and crushed, so as to obtain a splenic cell test sample in the form of fine small pieces. The obtained splenic cells were washed by centrifugation at 1000 rpm for 5 minutes using an RPMI-1640 medium. Meanwhile, myeloma cells (P3X63Ag8U1 cells) were cultured in advance under conditions of 5% CO₂, relative humidity of 100% and 37°C in an RPMI-1640 medium containing 10% FCS (fetal bovine serum), and the myeloma cells during the exponential growth phase were washed by centrifugation using an RPMI-1640 medium. The aforementioned splenic cells and the myeloma cells were mixed with each other, such that the ratio of the myeloma cells to the splenic cells was 4:1. The mixture cells were centrifuged at 1000 rpm for 5 minutes. The supernatant was discarded, and the cells were sufficiently loosened. To a centrifuge tube containing the cells, 1 mL of a solution consisting of 2 g of polyethylene glycol (M.W.1000, manufactured by Wako Pure Chemical Industries, Ltd.), 2 mL of an RPMI-1640 medium and 0.2 mL of DMSO (manufactured by NACALAI TESQUE, INC.) was gently added. The centrifuge tube was slowly rotated to mix the cells. One minute later, while the centrifuge tube was slowly rotated, 15 mL of an RPMI-1640 medium was added thereto taking three minutes. The cells were centrifuged at 1000 rpm for 5 minutes. Then, the supernatant was discarded, and the cells were sufficiently loosened. Thereafter, the cell concentration was adjusted to 1.6×10⁶ cells/mL in terms of the splenic cells using a HAT medium (manufactured by Gibco Corp.). The resulting cells were dispensed at a concentration of 0.2 mL/well into a 96-well microplate (manufactured by Sumitomo Bakelite Co., Ltd.). The cells were cultured under conditions of 5% CO₂, relative humidity of 100% and 37°C for approximately 1 to 2 weeks. After that, hybridomas grown in the wells were observed under a microscope.

### (1) Screening of Antibody of Interest

An antibody which binds to the PA4710 recombinant protein or to putative extracellular regions (SEQ ID NOS: 5 to 15) of the PA4710 protein was detected by the ELISA method described in Example 7. Moreover, an antibody to bind to the cell surface of the Pseudomonas aeruginosa was detected by the whole cell ELISA method described in Example 8.

### (2) Cloning of Cells Producing Antibody of Interest

As a result of the screening, the hybridomas that were determined to produce the antibody of interest were adjusted to 5 hybridomas/0.2 mL or 20 hybridomas/0.2 mL using a 10% FCS/HT (manufactured by Gibco Corp.) medium containing 5% BM-Condimed H1 Hybridoma Cloning Supplement (manufactured by Roche Diagnostics K. K.). The hybridomas were dispensed at a concentration of 0.2 mL/well of a 96-well microplate, followed by culturing. One to two weeks later, the growth of clones was observed under a microscope. The clones were analyzed by the method described in the section of screening to select clones producing the antibody of interest. Again, the hybridomas were adjusted to one hybridoma/0.2 mL or two hybridomas/0.2 mL using a 10% FCS/HT (manufactured by Gibco Corp.) medium containing 5% BM-Condimed H1 Hybridoma Cloning Supplement by the above-described method. Such hybridomas were dispensed at a concentration of 0.2 mL/well. One to two weeks later, the analysis was carried out by the method described in the section of screening to select monoclones producing the antibody of interest. Accordingly, obtained were the hybridomas under the accession numbers of FERM BP-10970, FERM BP-10971, FERM BP-10972, FERM BP-10973, and FERM BP-10974 at the National Institute of Advanced Industrial Science and Technology, International Patent Organism Depositary.

### (3) In Vitro Culture of Cells and Production of MAb

The clones of interest sufficiently proliferated in the 96-well microplate were scaled up gradually in a 48-well plate, a 12-well plate, a 50-mL flask, and a 250-mL flask, and cultured in a 10% FCS-RPMI medium. The cells obtained in this manner had MAb produced in the culture supernatant thereof, the MAb being detected by the ELISA method described in Example 7.

As a result, the absorbance in the ELISA for detecting the binding to the well, on which the synthetic peptide 4710L3A (SEQ ID NO: 26) of Example 5 including the extracellular region 4710Loop3 (SEQ ID NO: 7) was adsorbed, was 0.078 in the negative control 10% FCS-RPMI medium, whereas the absorbance of the culture supernatant of the hybridoma under the accession number of FERM BP-10971 at the National Institute of Advanced Industrial Science and Technology, International Patent Organism Depositary, was 1.382. The absorbance in the ELISA for detecting the binding to the well, on which the synthetic peptide 4710L7 (SEQ ID NO: 31) of Example 5 included in the extracellular region 4710Loop7 (SEQ ID NO: 11) was adsorbed, was 0.097 in the 10% FCS-RPMI medium, whereas the absorbance of the culture supernatant of the hybridoma under the accession number of FERM BP-10972 was 0.637. Moreover, the absorbance in the ELISA for detecting the binding to the well, on which the synthetic peptide 4710L8A (SEQ ID NO: 38) of Example 5 included in the extracellular region 4710Loop8 (SEQ ID NO: 12) was adsorbed, was 0.071 in the 10% FCS-RPMI medium, whereas the absorbance of the culture supernatant of the hybridoma under the accession number of FERM BP-10970 was 1.211. Furthermore, the absorbance of the culture supernatant of a hybridoma under the accession number of FERM P-21205 in the ELISA for detecting the binding to the well, on which the synthetic peptide 4710L8B (SEQ ID NO: 33) of Example 5 included in the extracellular region 4710Loop8 (SEQ ID NO: 12) was adsorbed, was 0.497, whereas the absorbance in the ELISA for detecting the binding to the well, on which the peptides other than 4710L8B (SEQ ID NO: 25 to SEQ ID NO: 32, and SEQ ID NO: 34 to SEQ ID NO: 39) were adsorbed, was 0.060 to 0.088. The absorbance of the culture supernatant of a hybridoma under the accession number of FERM BP-10974 in the ELISA for detecting the binding to the well, on which the synthetic peptide 4710L10 (SEQ ID NO: 35) of Example 5 included in the extracellular region 4710Loop10 (SEQ ID NO: 14) was adsorbed, was 0.810, whereas the absorbance in the ELISA for detecting the binding to the well, on which the peptides other than 4710L10 (SEQ ID NO: 25 to SEQ ID NO: 34, and SEQ ID NO: 36 to SEQ ID NO: 39) were adsorbed, was 0.061 to 0.085. From the result described above, it was demonstrated the MAb that binds to each peptide was produced.

### (4) In Vivo Cell Propagation in Ascites and Production of MAb

Each of the hybridomas under the accession numbers of FERM BP-10971, FERM BP-10972, FERM BP-10970, FERM BP-10973, and FERM BP-10974 at the National Institute of Advanced Industrial Science and Technology, International Patent Organism Depositary, was intraperitoneally administered in a BALB/c-nu/nu mouse (purchased from Charles River Laboratories Japan, Inc.) at a concentration of 1×10⁷/mouse. One to two week later, the ascites was collected. MAb included in the ascites was purified by the method described in Example 6. The obtained purified IgG fractions were designated as anti-4710L3A IgG (MAb), anti-4710L7 IgG (MAb), anti-4710L8A IgG (MAb), anti-4710L8B IgG (MAb), and anti-4710L10 IgG (MAb). The heavy chain and light chain of the IgG subclass of this rat MAb were determined by monoclonal antibody isotyping kit (RMT1, manufactured by Dainippon Pharmaceutical Co., Ltd.). As a result, it was determined that the heavy chains were IgG1, IgG2a, IgG1, IgG2b, and IgG2b, respectively, and that all the light chains were K.

The binding to the Pseudomonas aeruginosa surface of the mouse ascites-MAb purified by the method descried in Example 6 was confirmed by whole cell ELISA described in Example 8.

Result for anti-4710L7 IgG (MAb) : the absorbance of the anti-4710L7 IgG (MAb), which was the IgG fraction obtained by purifying the ascites of the mice to which the hybridoma under the accession number of FERM BP-10972 had been administered, was 0.601, whereas the absorbance of a negative control IgG fraction (50 µg/well) purified from reference rat sera obtained by administering only adjuvant was 0.280. This result demonstrated that the antibody (IgG) that recognizes the extracellular region of the PA4710 protein exposed from the cell surface was included in the IgG fraction.

Result for anti-4710L8B IgG (MAb): the absorbance of the anti-4710L8B IgG (MAb), which was the IgG fraction obtained by purifying the ascites of the mice to which the hybridoma under the accession number of FERM BP-10973 had been administered, was 0.530, whereas the absorbance of the negative control IgG fraction (50 µg/well) purified from reference rat sera obtained by administering only adjuvant was 0.244. This result demonstrated that the antibody (IgG) that recognizes the extracellular region of the PA4710 protein exposed from the cell surface was included in the IgG fraction.

Result for anti-4710L10 IgG (MAb): the absorbance of the anti-4710L10 IgG (MAb), which was the IgG fraction obtained by purifying the ascites of the mice to which the hybridoma under the accession number of FERM BP-10974 had been administered, was 0.435, whereas the absorbance of the negative control IgG fraction (50 µg/well) purified from reference rat sera obtained by administering only adjuvant was 0.092. This result demonstrated that the antibody (IgG) that recognizes the extracellular region of the PA4710 protein exposed from the cell surface was included in the IgG fraction.

### [Example 10] : Ability of PA4710 Recombinant Protein Immunized-Rat Sera to DefendAgainst PA103 Strain Systemic Infection in Normal Mice

In evaluation with systemically infected models of normal mice, living bacteria of the PA103 strain suspended in 500 µl of a saline containing 5% mucin were intraperitoneally administered to 4-week-old CD-1 mice (purchased from Charles River Laboratories Japan, Inc.) at a dose of 1.0×10⁵ cfu/mouse (20LD₅₀). Immediately thereafter, the serum sample 2.5 fold diluted with a saline was administered at a dose of 0.1 mL/mouse from the caudal vein. The protective activity against the infection was assessed based on survival after seven days.

As a result, 5 out of 7 mice died with negative control rat sham sera. By contrast, all mice survived in a group to which formalin-inactivated PA103 strain immunized-rabbit sera had been administered. Thus, the protective activity of the formalin-inactivated PA103 strain immunized-rabbit sera against the infection was confirmed. Under this condition, 6 out of 7 mice survived in a group to which the PA4710 recombinant protein immunized-rat sera had been administered. Thus, the protective activity of the PA4710 recombinant protein immunized-rat sera against the infection was confirmed.

### [Example 11]: Ability of PA4710 Monoclonal Antibody to Defend Against PA103 Strain Systemic Infection in Neutropenic Mice

In evaluation with systemically infected models of neutropenic mice, 12.5 mg/mL (saline) of cyclophosphamide (hereinafter referred to as CY. manufactured by Sigma-Aldrich Co.) was prepared and intraperitoneally administered to 4-week-old male CD-1 mice at three doses in total on day-5, -2, and 0 each at 125 mg/kg, so as to reduce the neutrophil level in the peripheral blood. Then, the PA103 strain suspended in 250 µl of a saline was intraperitoneally inoculated at a dose of 1.8×10⁵ cfu/mouse (133 LD₅₀). Immediately thereafter, each sample (sera and purified IgG fraction) diluted with a saline was administered at a dose of 0.2 mL/mouse from the caudal vein. The protective activity against the infection was assessed based on survival after seven days.

As a result, when the purified sera IgG fraction was used as the sample (0.5 mg/mouse), 6 out of 7 mice died in a group to which negative control rat sham IgG had been administered, and only one mouse survived. By contrast, 5 out of 7 mice survived in a group to which the anti-PA103 IgG obtained from the formalin-inactivated PA103 strain immunized-sera had been administered. Thus, the protective activity of the anti-PA103 IgG against the infection was confirmed. Under this condition, 4 and 5 out of 7 mice survived in the respective groups to which the anti-4710L7 IgG (MAb) and the anti-4710L8B IgG (MAb), which are the rat MAb obtained in Example 9, had been administered. Thus, the protective activity of the anti-4710L7 IgG and the anti-4710L8B IgG against the infection was confirmed.

### [Example 12] : Ability of Purified Sera IgG Fraction and Monoclonal Antibody to Defend Against Multidrug Resistant Pseudomonas aeruginosa Systemic Infection in Neutropenic Mice

The minimum growth inhibitory concentrations of various antibacterial agents against a multidrug resistant Pseudomonas aeruginosa MSC06120 strain were: 32 µg/ml for imipenem, 64 µg/ml for amikacin, and >256 µg/ml for ciprofloxacin. In evaluation with systemically infected models of neutropenic mice using this strain, 12.5 mg/mL (saline) of CY was prepared and intraperitoneally administered to 4-week-old male CD-1 mice at three doses in total on day-5, -2, and 0 each at 125 mg/kg, so as to reduce the neutrophil level in the peripheral blood. Then, the MSC06120 strain suspended in 250 µl of a saline was intraperitoneally inoculated at a dose of 1.73×10⁵ cfu/mouse (15.5 LD₅₀). Immediately thereafter, each sample diluted with a saline was administered at a dose of 0.2 mL/mouse from the caudal vein. The protective activity against the infection was assessed based on survival after seven days.

As a result, when the purified sera IgG fraction and the monoclonal antibody were used as the sample (0.5 mg/mouse), 4 out of 7 mice died in a group to which negative control rat sham IgG had been administered, and only three mice survived. By contrast, 4 out of 7 mice survived in a group to which the anti-PA103 IgG obtained from the formalin-inactivated PA103 strain immunized-sera had been administered. Under this condition, 6 and 5 out of 7 mice survived in the respective groups to which the anti-4710 IgG obtained in Example 6 and the anti-4710L10 IgG (MAb), which is the rat MAb obtained in Example 9, had been administered. Thus, the protective activity of the anti-4710 IgG and the anti-4710L10 IgG against the infection was confirmed.

### Industrial Applicability

The present invention is to provide a vaccine composition and a polyclonal antibody (hereinafter, referred to as PAb) or a monoclonal antibody (hereinafter, referred to as MAb) each of which has an ability to practically prevent or treat Pseudomonas aeruginosa infections, and furthermore which respond to the diversity of clinical isolates derived from patients of Pseudomonas aeruginosa infections. The present invention is applicable to a preventive agent, a therapeutic agent, or a diagnostic agent for Pseudomonas aeruginosa infections.

Furthermore, the antibody of the present invention binds to an extracellular region of a PA4710 protein, which is present in the outer membrane or outside of Pseudomonas aeruginosa. These regions are considered to be extremely highly conservative among the strains regardless of serotypes and the like. Thus, the antibody of the present invention react with various clinical isolates. Therefore, a high therapeutic effect on Pseudomonas aeruginosa infections is expected.

## Claims

1. A protein selected from the following (i), (ii), (iii), and (iv):
(i) a protein comprising the amino acid sequence represented by SEQ ID NO: 4;
(ii) a protein comprising an amino acid sequence in which one or more amino acids are deleted, substituted, inserted or added in the amino acid sequence represented by SEQ ID NO: 4, the protein being functionally equivalent to a protein consisting of the amino acid sequence represented by SEQ ID NO: 4;
(iii) a protein encoded by a polynucleotide which hybridizes under a stringent condition to a polynucleotide encoding the amino acid sequence represented by SEQ ID NO: 4, the protein being functionally equivalent to the protein consisting of the amino acid sequence represented by SEQ ID NO: 4; and
(iv) a protein comprising an amino acid sequence having 70% or more identity with the amino acid sequence represented by SEQ ID NO: 4, the protein being functionally equivalent to the protein consisting of amino acid sequence represented by SEQ ID NO: 4.

2. A peptide consisting of an amino acid sequence included in an amino acid sequence selected from the group consisting of positions 181 to 198, 204 to 257, 259 to 311, 313 to 319, 321 to 436, 440 to 491, 493 to 600, and 602 to 764 in the amino acid sequence represented by SEQ ID NO: 3, wherein
a peptide region exposed from a Pseudomonas aeruginosa surface is encoded.

3. A peptide consisting of an amino acid sequence in which one or a plurality of amino acids are conservatively substituted in the amino acid sequence of the peptide according to claim 2.

4. A peptide consisting of the amino acid sequence represented by any one of SEQ ID NOS: 5 to 15.

5. A peptide consisting of an amino acid sequence in which one or a plurality of amino acids are conservatively substituted in the amino acid sequence of the peptide according to claim 4.

6. A peptide consisting of at least 7 consecutive amino acids of the peptide according to claim 2.

7. A peptide consisting of at least 7 consecutive amino acids of the peptide according to claim 3.

8. A peptide consisting of at least 7 consecutive amino acids of the peptide according to claim 4.

9. A peptide consisting of at least 7 consecutive amino acids of the peptide according to claim 5.

10. An antibody or a functional fragment thereof, which is directed against a PA4710 protein or a portion thereof derived from Pseudomonas aeruginosa.

11. The antibody or the functional fragment thereof according to claim 10, wherein the portion of the PA4710 protein derived from Pseudomonas aeruginosa is a loop-containing cell surface region.

12. An antibody or a functional fragment thereof, which is directed against the protein according to claim 1.

13. An antibody or a functional fragment thereof, which is directed against the peptide according to claim 2.

14. An antibody or a functional fragment thereof, which is directed against the peptide according to claim 3.

15. An antibody or a functional fragment thereof, which is directed against the peptide according to claim 4.

16. An antibody or a functional fragment thereof, which is directed against the peptide according to claim 5.

17. An antibody or a functional fragment thereof, which is directed against the peptide according to claim 6.

18. An antibody or a functional fragment thereof, which binds to a peptide consisting of any one of the amino acid sequences of SEQ ID NOS: 5 to 15, but does not bind to a peptide consisting of any other amino acid sequence of SEQ ID NOS: 5 to 15.

19. The antibody or the functional fragment thereof according to claim 10, which binds to a surface of Pseudomonas aeruginosa.

20. The antibody or the functional fragment thereof according to any one of claims 10 to 19, wherein the antibody is a monoclonal antibody.

21. The antibody or the functional fragment thereof according to claim 10, which has an antibacterial activity in a patient infected with Pseudomonas aeruginosa.

22. The antibody or the functional fragment thereof according to claim 21, wherein the patient is a patient with a reduced neutrophil level.

23. The antibody or the functional fragment thereof according to claim 21, wherein the Pseudomonas aeruginosa is multidrug resistant Pseudomonas aeruginosa.

24. The antibody or the functional fragment thereof according to any one of claims 21 to 23, wherein the antibody is a monoclonal antibody.

25. An antibody or a functional fragment thereof, which is produced by a hybridoma deposited under any one of accession numbers FERM BP-10970, FERM BP-10971, FERM BP-10972, FERM BP-10973, and FERM BP-10974.

26. A monoclonal antibody or a functional fragment thereof, which reacts with an antigen identical to an antigen of a monoclonal antibody produced by a hybridoma deposited under any one of accession numbers FERM BP-10970, FERM BP-10971, FERM BP-10972, FERMB P-10973, and FERM BP-10974.

27. A hybridoma producing the antibody according to claim 20.

28. A hybridoma producing the antibody according to claim 24.

29. A hybridoma deposited under any of accession numbers FERM BP-10970, FERM BP-10971, FERM BP-10972, FERM BP-10973, and FERM BP-10974.

30. An antigen composition comprising any one of a protein antigen and a peptide antigen which are capable of inducing production of an antibody directed against a PA4710 protein derived from Pseudomonas aeruginosa.

31. An antigen composition comprising any one of the protein according to claim 1 and the peptide according to any one of claims 2 to 9.

32. A vaccine composition for use in prevention or treatment of a disease associated with Pseudomonas aeruginosa, the vaccine composition comprising the antigen composition according to any one of claims 30 and 31 and optionally comprising at least one pharmaceutically acceptable carrier, diluent and/or adjuvant.

33. The vaccine composition according to claim 32, wherein the disease associated with Pseudomonas aeruginosa is a systemic infectious disease caused by a Pseudomonas aeruginosa infection

34. The vaccine composition according to claim 33, wherein the Pseudomonas aeruginosa infection is a multidrug resistant Pseudomonas aeruginosa infection.

35. A pharmaceutical composition for use in prevention or treatment of a disease associated with Pseudomonas aeruginosa, the pharmaceutical composition comprising the antibody or the functional fragment thereof according to any one of claims 10 to 19, 21 to 23, 25, and 26 and optionally comprising at least one pharmaceutically acceptable carrier and/or diluent.

36. The pharmaceutical composition according to claim 35, wherein the disease associated with Pseudomonas aeruginosa is a systemic infectious disease caused by a Pseudomonas aeruginosa infection.

37. The pharmaceutical composition according to claim 36, wherein the Pseudomonas aeruginosa infection is a multidrug resistant Pseudomonas aeruginosa infection.

38. A diagnostic agent for a Pseudomonas aeruginosa infection, comprising the antibody or the functional fragment thereof according to any one of claims 10 to 19, 25, and 26.

39. A detection kit for Pseudomonas aeruginosa, comprising the antibody or the functional fragment thereof according to any one of claims 10 to 19, 25, and 26.
